# EUROPEAN PATENT APPLICATION

(11) **EP 4 174 075 A1**
(43) Date of publication of application: **03.05.2023**
(21) Application number: 21382981.5
(22) Date of filing: 29.10.2021
(51) Int. Cl.: C07D 498/04, A61K 31/4355, A61K 31/7056, C07H 19/044, A61P 37/00, A61P 37/02, A61P 37/04, A61P 37/06, A61P 37/08, A61P 29/00, A61P 1/00, A61P 1/16, A61P 3/00, A61P 31/00, A61P 11/00, A61P 11/06

(54) **5-AMINOHEXAHYDRO-6,7,8-TRIHYDROXY-3H-OXAZOLO[3,4-A]PYRIDIN-3-ONE DERIVATIVES DERIVATIVES AS TLR4 MODULATORS FOR THE TREATMENT OF IMMUNE DISEASES**

(71) Applicant: Consejo Superior de Investigaciones Científicas (CSIC), 41013 Sevilla (ES); Universidad de Sevilla, 41013 Sevilla (ES); Academia Sinica, Taipei 11529 (TW)
(72) Inventor: García Fernandez, José Manuel, 41013 Sevilla (ES); Ortiz Mellet, Carmen, 41013 Sevilla (ES); González Cuesta, Manuel, 41013 Sevilla (ES); Sánchez Fernández, Elena Matilde, 41013 Sevilla (ES); Chang, Ya-Jen, 115 Taiwan (TW); Lai, Alan Chuan-Ying, 115 Taiwan (TW)
(74) Representative: Pons

(57) **Abstract**

The invention relates to a 5-aminohexahydro-6,7,8-trihydroxy-3H-oxazolo[3,4-a]pyridin-3-one (i.e. 2,3,4-trihydroxy-5N,6O-oxomethylidene-nojirimycin-1-amine) glycolipid mimetic derivative of formula **I** 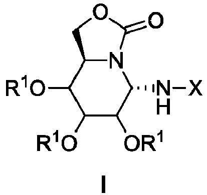 acting as antagonists or agonist of the TLR4 for the treatment and/or prevention of an immune disease such as e.g. acute inflammation, chronic disease allergy, cancer chemotherapy, infectious disease, the Metabolic Syndrome, non-alcoholic fatty liver disease (NAFLD), non-alcoholic steatohepatitis (NASH), immune mediated hepatitis, an autoimmune disease, graft rejection pathology, inflammatory bowel disease, atherosclerosis and airway hyperactivity, such as e.g. asthma and allergic rhinitis.
An exemplary compound is e.g. example 1, compound 1:

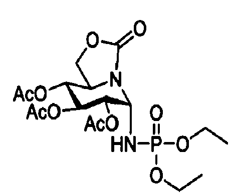

## Description

The invention relates to a glycolipid mimetic derivative of formula I, wherein X and R¹ are defined in the description, or a pharmaceutical composition thereof and its use as for the treatment and/or prevention of an immune disease acting as antagonists or agonist of the TLR4.

### BACKGROUND ART

Inflammation is a physiological response of the immune system to injury and infection. This process activates signaling routes aiming at healing and repairing damaged tissue, as well as at defending itself against infective agents, such as viruses and bacteria. However, unresolved or inappropriately activated inflammation can become pathogenic. Thus, chronic inflammation is the primary cause of a broad spectrum of diseases, comprising, among others, rheumatoid arthritis (RA), inflammatory bowel disease (IBD; including gastrointestinal conditions such as Crohn's disease and ulcerative colitis), chronic obstructive pulmonary disease (COPD), asthma, psoriasis and idiopathic pulmonary fibrosis (IPF). On the other hand, viral and bacterial infections or other hostile agents (such as toxins, chemicals and so forth) can lead to uncontrolled acute inflammatory responses and injury often seen in patients with underlying pathogenic conditions (such as COPD or asthma). A recent example of virally induced inflammation leading to acute lung injury and acute respiratory distress syndrome includes SARS-CoV-2 infection, which is associated with a cytokine storm characterized by high levels of IL-6, IL-12 and IL-1β, and tumor necrosis factor (TNF) and defective type I interferon activity. Although a battery of biologics and chemotherapeutics are effective in treating various inflammatory diseases, a large proportion of patients are not responsive to current therapies, highlighting the need for new treatment approaches. Lack of target specificity and side effects are the current problems hampering clinical application of small molecule anti-inflammatory drugs. Discovery of novel target-specific compounds for treatment of these diseases is a big challenge with potentially significant scientific, commercial and social impacts. For a general perspective of inflammation, including mechanisms at play, inflammation-associated pathologies, current anti-inflammatory therapies and remaining challenges, see: Innate Immunity 2021, 27, 275-284; Pharmaceutics 2021, 13, 64; Nature 2008, 454, 24.

Carbohydrates play crucial roles in the immune system function and the regulation of the immune response, and a variety of natural carbohydrate-based immunomodulators have been clinically evaluated for applications as anti-inflammatory drugs. However, immunoactive carbohydrates obtained from natural sources are usually difficult to obtain in sufficient quantity, purity and homogeneity. Synthetic organic chemistry, including total synthesis and semi-synthetic strategies, offers a more attractive approach to molecularly defined, improved versions of carbohydrate-containing anti-inflammatories, enabling structural modification of the corresponding natural products with a high level of chemical control. In this context, glycolipid derivatives behaving as antagonist of the toll-like receptor 4 (TLR4) represent an appealing option. For selected review articles on carbohydrate derivatives, especially glycolipids, as immunomodulators, see: Org. Biomol. Chem., 2011, 9, 3080-3104; Molecules 2013, 18, 15662-15688; Clin. Transl. Immunol. 2016, 5, e69; Chem. Eur. J. 2017, 23, 1728-1742; Nat. Rev. Chem. 2021, 5, 197-216. TLR4 belongs to the pattern recognition receptor family, which plays a key role in the human defense mechanism and responds to invading pathogens with high selectivity and sensitivity. TLR4 is sensitive to pathogen-associated molecular patterns (PAMPs) such as lipopolysaccharide (LPS) and lipo-oligosaccharide (LOS). Moreover, TLR4 recognizes PAMPs from fungi, viruses, and mycoplasmas. In addition to PAMPs, TLR4 can be activated by certain endogenous ligands produced due to tissue injury and/or inflammation. This receptor-ligand interaction initiates an intracellular signaling cascade that leads to the subsequent proinflammatory response. Due to the involvement of TLR4 in various pathological conditions coursing with inflammation, compounds antagonizing TLR4 by acting on the TLR4-dependent signaling cascade are potential anti-inflammatory candidates. For selected review articles on TLR4 and the interest of TLR4 antagonists as anti-inflammatories, see: Molecules 2020, 25, 627; Front. Immunol. 2020, 11, 1210; Nat. Rev. Drug Discov. 2021, 20, 39.

Interestingly, anti-inflammatory TLR4 antagonists can be further used in the formulation of vaccine adjuvants. As an example, it has been shown that monophosphoryl lipid A (MPL^{®}), a vaccine adjuvant manufactured from a *Salmonella enterica* endotoxin that has achieved widespread clinical and market acceptance, contains two structural variants that functioned as competitive antagonists of human TLR4 (see: Front. Immunol. 2020, 11, 577823). A partial agonist profile, necessary for the adjuvant behavior, could be recapitulated and manipulated by spiking synthetic agonists with synthetic antagonists to achieve a broad dose range over which TLR4 stimulation could be constrained below a desired threshold. Additionally, it should be considered that compounds acting on the TLR4 signaling pathway can eventually develop antagonistic or agonistic activity in a context-dependent manner, enabling, e.g., the treatment of an inflammatory pathology and promoting an enhanced immune response towards a given antigen, thus behaving as an adjuvant.

A few glycolipids targeting the TLR4 route already reached clinical trials. The iconic example of TLR4 antagonist with anti-inflammatory properties is eritoran (E5564), an underacylated lipid A analog. Eritoran was being evaluated for treatment of sepsis, but unfortunately could not meet its target end-point in phase 3. Moreover, the complexity of the chemical structure, particularly the presence of two labile anionic phosphate groups in the amino disaccharide core of eritoran as well as in other lipid A analogs, makes their synthesis rather cumbersome and represents a serious hurdle for optimization strategies. For relevant references at this respect, see: J. Am. Chem. Soc. 2019, 141, 947-9478; Med. Res. Rev. 2019, 39, 1053-1090; Future Med. Chem. 2018, 10, 461-476.

Neutral trivalent α-D-mannopyranoside derivatives bearing a long acyl lipid chain have also been found to behave as TLR4 antagonists, as reported in ACS Chem. Biol. 2015,10, 2697-2705. The enzymatic lability of the *O*-glycosidic linkage makes these compounds unstable in biological media and have not been pursued to clinical trials. More recently, metabolically stable glycolipid mimetics entailing a monosaccharide-like polysubstituted 2-oxa-3-oxoindolizidin-5-yl radical glycone moiety linked through a sulfur (S) atom to a lipid aglycone were reported to elicit an anti-inflammatory response in LPS-exposed immune system cells, such as microglia and dendritic cells, as well as in a mouse model of acute inflammation. The glycone moiety in these compounds belong to the so-called sp²-iminosugar glycomimetic family, and the corresponding conjugates with a lipid aglycone are termed sp²-iminosugar glycolipids. Mechanistic studies confirmed that this type of compound act on the TLR4 inflammatory cascade, leading to the modulation of the phosphorylation state of mitogen activated protein kinases (MAPKs) such as JNK and p38α and to a reduction in the levels of proinflammatory cytokines and reactive oxygen species (ROS). Concomitantly, they enhance the expression of anti-inflammatory markers. The narrow range of sulfur functionalities showing the desired activity, namely sulfone and sulfoxide, and the fact that the latter are obtained as mixtures of the (S_{S}) and (S_{R}) diastereomers that need to be separated, is a main limitation. For relevant references on this type of S-linked glycolipid mimetics as anti-inflammatories, see: Eur. J. Med. Chem. 2019, 169, 111-120; Food Chem. Toxicol. 2018, 111, 454-466; Biochim. Biophys. Acta 2016, 1862, 1663-1674

Replacement of the pseudo anomeric sulfur by an amine (NH) group in sp²-iminosugar glycolipid mimetics has been explored, but only the *N*-glycolipid mimetics having polyfluoroalkyl segments were found to exhibit anti-inflammatory activity, as reported in Eur. J. Med. Chem. 2019, 182, 11160. Moreover, the basic character of the amine group makes these compounds sensitive to pH. Most worrying, amine-linked sp²-iminosugar conjugates have been reported to behave as inhibitors of various glycosidases, which might result in unwanted side effects. For relevant references at this respect, see: Chem. Commun., 2010, 46, 5328-5330; Carbohydr. Res. 2016, 429, 113-122. Thus, the search for metabolically stable glycolipid mimetics with specific TLR4 antagonistic activity, accessible through efficient synthetic methodologies, for use as anti-inflammatory drugs is still in progress.

Pseudoamide derivatives, that is, derivatives incorporating an achiral functional group containing a N-X(=Y)- segment, where X is a carbon (C), sulfur (S) or phosphorous atom (P) with, at least, one double bond, and Y is oxygen (O), sulfur, selenium (Se) or nitrogen (N), are characterized by high chemical and enzymatic stability and are amenable to efficient synthesis. A previous patent document, namely Patent ES 2 370 852 B1, reports a limited number of examples of derivatives where a sp²-iminosugar glycone moiety was connected to an aglycone moiety through a pseudoamide group, specifically through a urea, a thiourea or a guanidine group. The said compounds were claimed to act as inhibitors of the α-glucosidases and be useful for the treatment of a disease mediated by the α-glucosidases. As selected examples, cancer, viral infections, tuberculosis, diabetes and glycosphingolipid storage disorders were mentioned. However, the said patent document does not claim the use of the compounds as anti-inflammatories, nor provide evidences of anti-inflammatory activities of the compounds, nor the information provided in the cited patent document let infer that the compounds could exhibit anti-inflammatory properties and be used as anti-inflammatories for the treatment of inflammation-related disease.

Further patent (e.g., WO 2013/054070 Al) and non-patent documents (e.g., ACS Omega 2020, 5, 16263-16271) report iminosugar-type glycomimetics with anti-inflammatory activity in specific contexts. None of such glycomimetics contains the 2-oxa-3-oxoindolizidine core, nor they incorporate a lipid moiety. As in the above-mentioned art, their biological activity is related to their ability to inhibit certain glycosidases. No evidence of a mechanism of action implying TLR4 antagonism or agonism is shown.

In view of the above, it would be useful to identify additional glycolipid-like agents that can specifically induce an anti-inflammatory response or modulate the immune response by antagonizing or agonizing the TLR4 receptor. Of particular interest is developing new strategies allowing accessing such compounds with high efficiency while warranting: (a) total stereoselectivity during the generation of the glycosidic linkage, (b) metabolic stability and (c) compatibility with molecular diversity-oriented strategies, thus permitting systematic modifications of the structure to optimize the immunomodulatory profile towards an anti-inflammatory response.

### SUMMARY OF THE INVENTION

The present invention discloses a novel glycolipid mimetic that is metabolically stable and can be selectively modified to optimize the immunomodulatory profile in view of promoting an anti-inflammatory or an adjuvant response. The present invention also discloses novel glycolipid mimetics that are able to express anti-inflammatory and adjuvant activity in a context dependent manner. The present invention also provides a process to obtain said glycolipid mimetic with total stereoselectivity. Further on, the present invention provides a medicament such as an anti-inflammatory agent or a vaccine containing a glycolipid mimetic.

According to present invention, a compound wherein (i) the sugar-like glycone portion which is part of the glycolipid mimetic is a polysubstituted 2-oxa-3-oxoindolizidin-5-yl radical, (ii) this radical is linked to the lipid aglycone moiety through an achiral pseudoamide functional group and (iii) the said glycone radical is directly attached to a nitrogen atom of the pseudoamide group, selectively induces production of cytokines associated to an anti-inflammatory response. The present invention discloses a process for preparing a compound according to the invention with total α-anomeric stereoselectivity and is not degraded by the α-glycosidases that hydrolyze the glycosidic linkage in the natural or synthetic α-glycolipids, which warrants a higher metabolic stability. Further, present invention discloses a compound according to the invention useful for the modulation of the immune system toward anti-inflammatory cytokine producing cells. Accordingly, a compound according to present invention can be used in the treatment and/or prevention of inflammation-associated conditions.

A first aspect of the present invention relates to a compound of formula I: 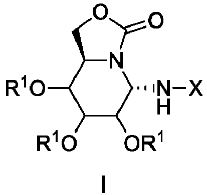 wherein:
each R¹ is independently -H, -CH₂Ph (Bn), -COPh (Bz), -CO-C₁-C₁₂ alkyl, C₁-C₁₂ alkyl,
wherein each R¹ is independently optionally substituted by one or more groups R²;
R² is -OH, -N(R³)₂, -N₃, C₁-C₄ alkyl or halogen;
each R³ is independently H or C₁-C₄ alkyl;
X is -C(=S)-OY, -C(=Se)-NHY, -SO₂-NYY', -SO₂-OY or -P(=O)(OY)₂;
Y is -C₁-C₂₅ alkyl optionally substituted by one or more groups R⁴;
Y' is H or -C₁-C₂₅ alkyl optionally substituted by one or more groups R⁴;
R⁴ is -OH, OBn, C₁-C₁₂ alkyl, -O-C₁-C₁₂ alkyl, -S-C₁-C₁₂ alkyl, -N(R³)₂, -N₃, -C(=O)- C₁-C₁₂ alkyl or Cy₁; and
Cy₁ is a 5-membered to 14-membered ring which can be saturated, partially unsaturated or aromatic, and which optionally contains from 1 to 4 identical or different heteroatoms in total selected from N, O, S or P, wherein Cy₁ can be optionally fused to a 5- or 6-membered carbocycle or heterocycle which can be saturated, partially unsaturated or aromatic, and wherein Cy₁ is optionally substituted by one or more groups selected from phenyl, halogen, C₁-C₄ alkyl, halo-C₁-C₄alkyl, -O-C₁-C₄alkyl, - OCOO-C₁-C₄ alkyl, -CO-C₁-C₄ alkyl, S-C1-C4 alky or N₃-C₁-C₄ alkyl.

In another embodiment the invention relates to a compound of formula **I** as defined above wherein each R¹ is independently selected from -H, -Bn, -Bz, -CO-C₁-C₄ alkyl and -C₁-C₄ alkyl and wherein each R¹ is independently optionally substituted by one or more groups R², preferably wherein each R¹ is independently selected from -H, -Bn, -Bz, -CO-CH₃ (Ac) and -C₁-C₄ alky and wherein each R¹ is independently optionally substituted by one or more groups R²; more preferably wherein each R¹ is independently selected from -H, Bn, Bz, Ac and -C₁-C₄ alkyl; and still more preferably wherein each R¹ is independently selected from -H and Ac.

In another embodiment, the invention relates to a compound of formula **I** as defined above wherein X is selected from -C(=S)-OY, -C(=Se)-NHY, -SO₂-NYY', -SO₂-OY or -P(=O)(OY)₂; preferably wherein X is selected from -C(=Se)-NYY', -SO₂-NYY', -SO₂-OY or -P(=O)(OY)₂; more preferably wherein X is selected from -C(=Se)-NHY', -SO₂-NYY' or -SO₂-OY, and still more preferably wherein X is -C(=Se)-NHY' or -SO₂-NYY'.

In another embodiment the invention relates to a compound of formula **I** as defined above wherein Y is -C₂-C₁₈ alkyl optionally substituted by one or more groups R⁴; and preferably wherein Y is -C₂-C₁₈ alkyl.

In another embodiment the invention relates to a compound of formula **I** as defined above, wherein Y' is H or -C₂-C₁₈ alkyl optionally substituted by one or more groups R⁴, and preferably wherein Y' is H.

In another embodiment, the invention relates to a compound of formula **I** as defined above wherein X is selected from -C(=S)-OY, -C(=Se)-NHY, -SO₂-NYY', -SO₂-OY or -P(=O)(OY)₂; preferably wherein X is selected from -C(=Se)-NYY', -SO₂-NYY', -SO₂-OY or -P(=O)(OY)₂; more preferably wherein X is selected from -C(=Se)-NHY', -SO₂-NYY' or -SO₂-OY, and still more preferably wherein X is -C(=Se)-NHY' or -SO₂-NYY'; Y is -C₂-C₁₈ alkyl optionally substituted by one or more groups R⁴ and preferably wherein Y is -C₂-C₁₈ alkyl; and
Y' is H or -C₂-C₁₈ alkyl optionally substituted by one or more groups R⁴, and preferably wherein Y' is H.

In another embodiment, the compound of formula **I** as defined above is selected from:
**1** 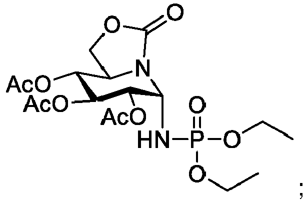
**2** 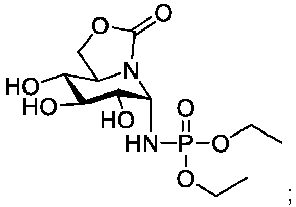
**3** 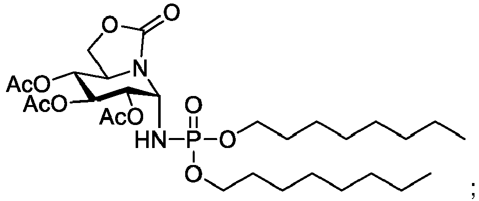
**4** 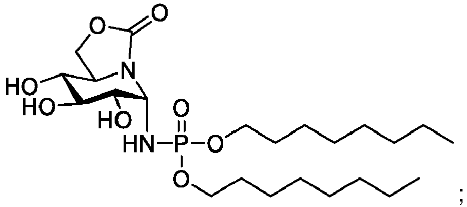
**5** 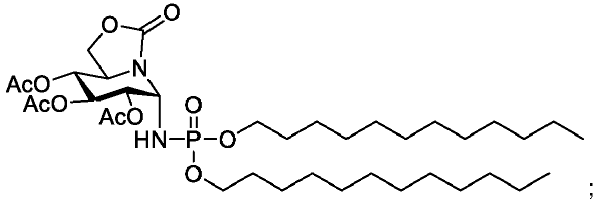
**6** 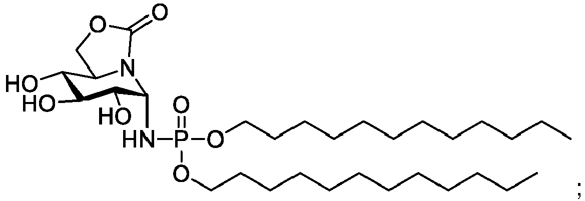
**7** 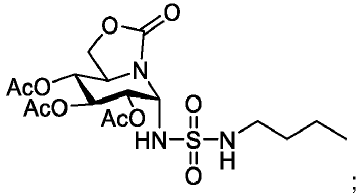
**8** 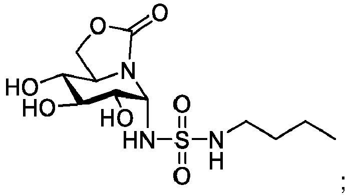
**9** 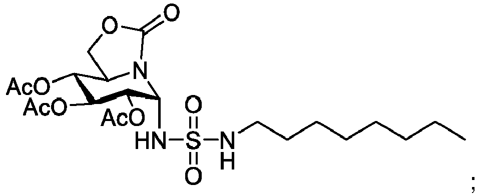
**10** 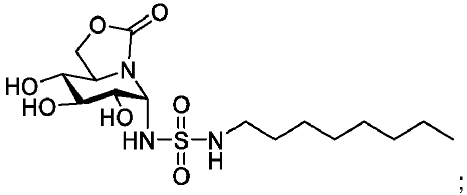
**11** 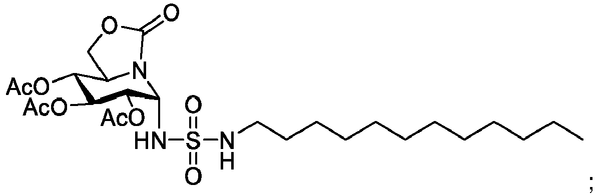
**12** 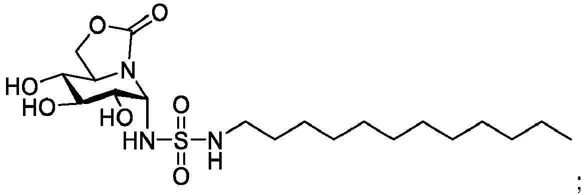
**13** 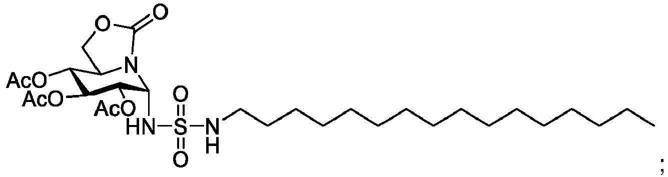
**14** 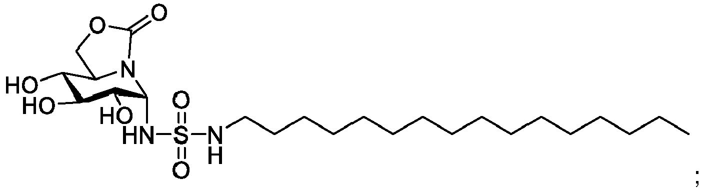
**15** 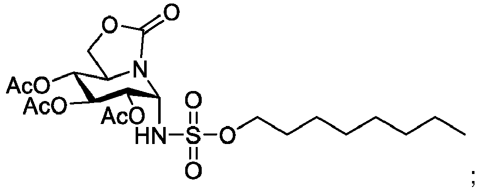
**16** 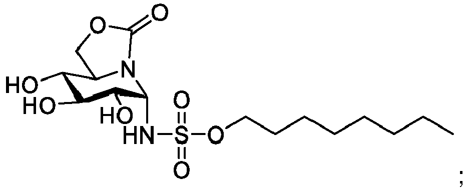
**17** 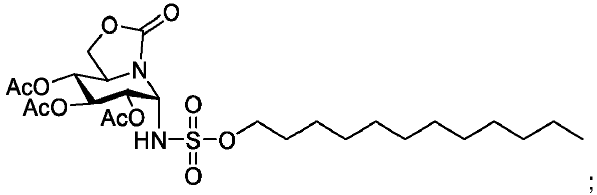
**18** 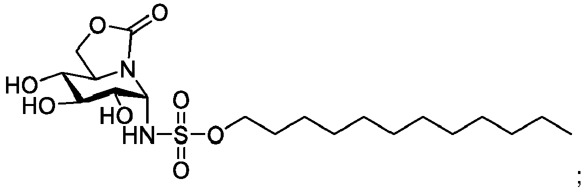
**19** 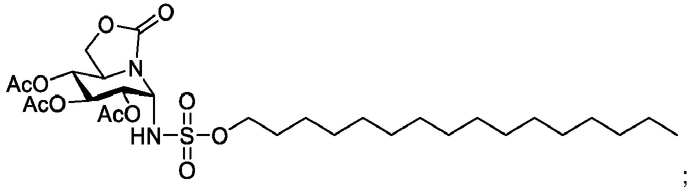
**20** 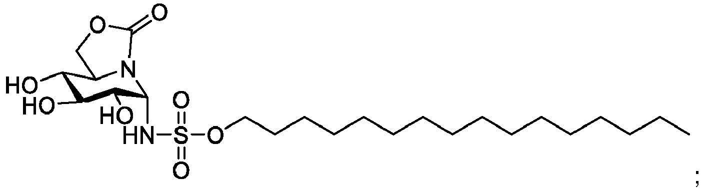
**24** 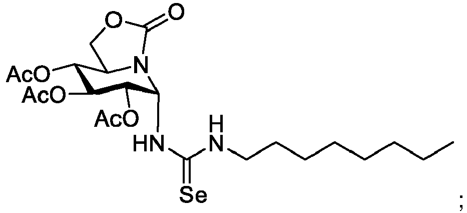
**28** 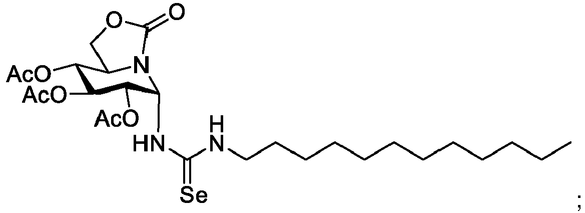
**29** 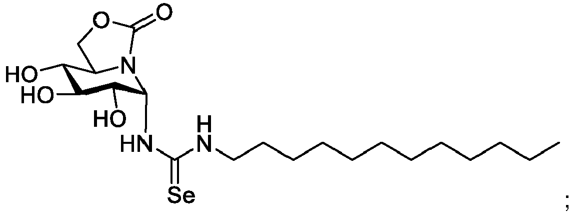
**31** 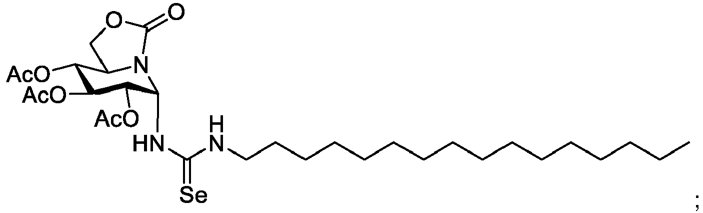
**32** 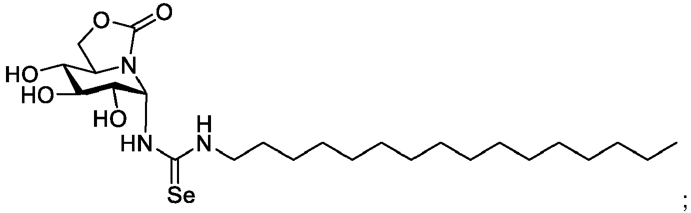
**33** 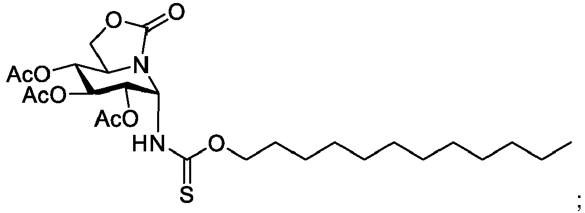 and
**34**

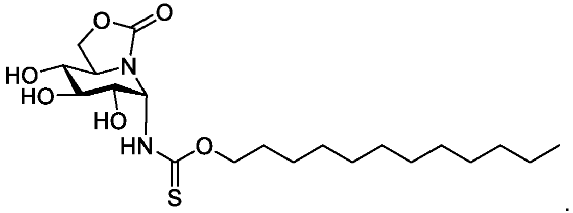

In some embodiments, any configurational pattern of the polysubstituted 2-oxa-3-oxoindolizidin-5-yl moiety (A) and anyone of the substituents R¹ and X formula **I** may independently be combined, *i.e.* individual substituents may be selected from the multiple compounds as if each specific combination was explicitly listed.

As used herein the singular forms "a", "and", and "the" include plural referents unless the context clearly dictates otherwise. For example, "a compound" refers to one or more of such compounds as known to those skilled in the art.

Throughout this application, it is contemplated that the term "compound" or "compounds" refers to the compounds discussed herein and includes precursors and derivatives of the compounds, including acyl-protected derivatives, and pharmaceutically acceptable salts of the compounds, precursors, and derivatives.

The compounds of formula **I** of the present invention are capable of acting as antagonists or antagonists of the TLR4 receptor in immune system cells. In yet another aspect, the compounds of the present invention are capable of eliciting an anti-inflammatory response. In another implementation, the compounds of formula **I** of the invention are capable of counterbalance an inflammatory response elicited by a third agent. In an exemplary implementation, the compounds of formula **I** of the invention are capable of acting as antagonists or agonists of the TLR4 receptor in immune system cells in vitro. In another implementation, the compounds of formula **I** of the invention are capable of acting as antagonists or agonists of the TLR4 receptor in immune system cells in vivo.

The invention also includes prodrugs of the compounds, pharmaceutical compositions including the compounds and a pharmaceutically acceptable carrier, and pharmaceutical compositions including prodrugs of the compounds and a pharmaceutically acceptable carrier.

The compounds of formula **I** of the present invention may contain one or more asymmetric centers and can thus occur as racemates and racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. Any formulas, structures or names of compounds described in this specification that do not specify a particular stereochemistry are meant to encompass any and all existing isomers as described above and mixtures thereof in any proportion. When stereochemistry is specified, the invention is meant to encompass that particular isomer in pure form or as part of a mixture with other isomers in any proportion.

Accordingly, the compounds of formula **I** of the invention may contain chiral centers or double bonds, In the case that contains chiral centers, those may indistinctly have the (*R*) or (*S*) configuration. In the case that contains double bonds, those may indistinctly have the (*Z*) or (*E*) configuration.

Along the present invention the term "pseudoamide group" refers to a nitrogen-containing functional group encompassing a N-X(=Y)- segment, where X is a carbon (C), sulfur (S) or phosphorous atom (P) with, at least, one double bond, and Y is oxygen (O), sulfur (S), selenium (Se) or nitrogen (N).

The terms "C₁-C₄, C₁-C₁₂, C₁-C₂₅ and C₂-C₁₈ alkyl", as a group or part of a group, means a straight or branched alkyl chain which contains from 1 to 4, from 1 to 12, from 1 to 25 or from 2 to 18 carbon atoms, respectively, and may contain unsaturations. "Unsaturations" may consist in double or triple bonds.

"Halogen" means fluoro, chloro, bromo or iodo.

The term Cy₁ refers to a 5-membered to 14-membered ring which can be saturated, partially unsaturated or aromatic, and which optionally contains from 1 to 4 identical or different heteroatoms in total selected from N, O, S or P, wherein Cy₁ can be optionally fused to a 5- or 6-membered carbocycle or heterocycle which can be saturated, partially unsaturated or aromatic, and wherein Cy₁ is optionally substituted by one or more groups selected from phenyl, halogen, C₁-C₄ alkyl, halo-C₁-C₄ alkyl, - O-C₁-C₄ alkyl, -OCOO-C₁-C₄ alkyl, -CO-C₁-C₄ alkyl, S- C₁-C₄ alky or N₃-C₁-C₄ alkyl. Examples include among others aziridine, oxirane, thiirane, azetidine,oxetane, thietane, azolidine, oxolane, thiolane, phopholane, piperidine, piperazine tetrahydropyrane, oxane, 1,4-dioxane, morpholine, phosphinane, phenyl, biphenyl, naphthyl, indanyl, indenyl, tetrahydronaphthyl, 2,3-dihydrobenzofuranyl, dihydrobenzopyranyl, 1,4-benzodioxanyl, furan, thiophene, pyrrole, oxazole, thiazole, imidazole, pyrazole, isoxazole, isothiazole, 1,2,3-oxadiazole, 1,2,3-triazole, 1,2,4-triazole, 1,3,4-thiadiazole, tetrazole, pyridine, pyridazine, pyrimidine, pyrazine, 1,3,5-triazine, imidazole, benzimidazole, benzoxazole, benzothiazole, indolizine, indole, isoindole, benzofuran, benzothiophene, 1H-indazole, purine, 4Hquinolizine, quinoline, isoquinoline, cinnoline, phthalazine, quinazoline, quinoxaline, 1,8-naphthyridine and pteridine.

When in the definitions used throughout the present specification for cyclic groups Cy₁ the said cyclic group refers to a radical of a ring in general terms, for example pyridyl, thienyl or indolyl, all the available bonding positions are included. Thus, for example, in the definitions of Cy₁, which do not include any limitation regarding the bonding position, the term pyridyl includes 2-pyridyl, 3-pyridyl and 4-pyridyl; thienyl includes 2-thienyl and 3-thienyl; and indolyl includes 1-indolyl, 2-indolyl, 3-indolyl, 4-indolyl, 5-indolyl, 6-indolyl and 7-indolyl.

"Optional" or "optionally" means that the subsequently described event of circumstances may or may not occur, and that the description includes instances where said event or circumstance occurs one or more times and instances in which it does not. For example, "optionally substituted" means that the referred group may or may not be substituted and that the description includes both substituted groups and groups having no substitution, and the substitution may occur one or more times.

The compound of the present invention can be prepared by various methods that involve the reaction of a suitable polysubstituted 2-oxa-3-oxoindolizidine derivative bearing a reactive group at position C-5 with an appropriate precursor of the lipid portion to generate the pseudoamide-type linkage. This reaction is termed the conjugation step.

Another aspect of the invention refers to a process for preparing a compound of formula **I** wherein X is -C(=S)-OY, which comprises the reaction of a compound of formula **II** and a compound of formula Y-OH: 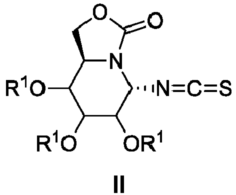 wherein R¹ and Y have the meaning of a compound of formula **I** wherein X is -C(=S)-OY.

Another aspect of the invention relates to a process for preparing a compound of formula **I** wherein X is -C(=Se)-NHY which comprises the reaction of a compound of formula **III** with LiAIHSeH: 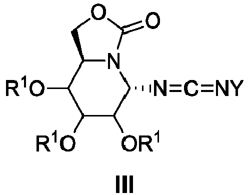 wherein R¹ and Y have the meaning of a compound of formula I wherein X is -C(=Se)-NHY.

Another aspect of the invention refers to a process for preparing a compound of formula **I** wherein X is selected from -SO₂-NYY' or -SO₂-OY which comprises the reaction of a compound of formula **IV** and a compound of formula H₂NS(=O)₂R², H₂NS(=O)₂NYY' or H₂NS(=O)₂OR² in the presence of a glycosidation promotor, and preferably in presence of boron trifluoride etherate (BF₃-Et₂O): 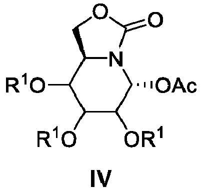 wherein R¹, Y and Y' have the meaning of a compound of formula **I** wherein X is -SO₂-NYY' or -SO₂-OY.

Another aspect of the invention relates to a process for preparing a compound of formula **I** wherein X is P(=O)(OY)₂ which comprises the reaction of a compound of formula **V** with a compound of formula P(=O)(OY)₃: 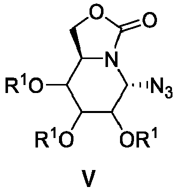 wherein R¹ and Y have the meaning of a compound of formula **I** wherein X is P(=O)(OY)₂.

Another aspect of the invention relates to a pharmaceutical composition which comprises at least one compound of formula **I** or any mixtures or combinations thereof.

The composition of the invention my optionally comprise at least one of a pharmaceutically acceptable carrier, diluent, excipient and/or additive.

Another aspect of the invention relates to a compound of formula **I** or a pharmaceutical composition thereof as defined above for use as a medicament.

Another aspect of the invention relates to the use of a compound of formula I as defined above or a pharmaceutical composition thereof for the manufacture of a medicament.

Another aspect of the invention relates to a method of treatment or prevention of a disease in a subject in need, especially a human being, which comprises administering to the subject in need an effective amount of a compound of formula I as defined above or a pharmaceutical composition thereof.

Another aspect of the invention relates to a compound of formula **I**': 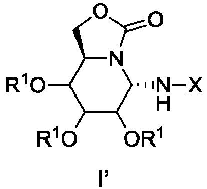 wherein:
each R¹ is independently -H, -CH₂Ph (Bn), -COPh (Bz), -CO-C₁-C₁₂ alkyl, C₁-C₁₂ alkyl,
wherein each R¹ is independently optionally substituted by one or more groups R²;
R² is -OH, -N(R³)₂, -N₃, C₁-C₄ alkyl or halogen;
each R³ is independently H or C₁-C₄ alkyl;
X is -C(=S)-OY, -C(=S)-NYY', -C(=O)-NYY', C(=Se)-NHY, -C(=NY)-NHY', -SO₂-Y, - SO₂-NYY', -SO₂-OY or -P(=O)(OY)₂;
Y is -C₁-C₂₅ alkyl optionally substituted by one or more groups R⁴;
Y' is H or -C₁-C₂₅ alkyl optionally substituted by one or more groups R⁴;
R⁴ is -OH, OBn, C₁-C₁₂ alkyl, -O-C₁-C₁₂ alkyl, -S-C₁-C₁₂ alkyl, -N(R³)₂, -N₃, -C(=O)- C₁-C₁₂ alkyl or Cy₁; and
Cy₁ is a 5-membered to 14-membered ring which can be saturated, partially unsaturated or aromatic, and which optionally contains from 1 to 4 identical or different heteroatoms in total selected from N, O, S or P, wherein Cy₁ can be optionally fused to a 5- or 6-membered carbocycle or heterocycle which can be saturated, partially unsaturated or aromatic, and wherein Cy₁ is optionally substituted by one or more groups selected from phenyl, halogen, C₁-C₄ alkyl, halo-C₁-C₄alkyl, -O-C₁-C₄alkyl, - OCOO-C₁-C₄ alkyl, -CO-C₁-C₄ alkyl, S-C1-C4 alky or N₃-C₁-C₄ alkyl,
or a pharmaceutical composition thereof for use in the treatment and/or prevention of an immune disease.

Another aspect of the invention relates to the use of a compound of formula **I'** as defined above or a pharmaceutical composition thereof for the treatment and/or prevention of an immune disease.

Another aspect of the invention relates to a method of treatment and/or prevention of an immune disease in a subject in need, especially a human being, which comprises administering to the subject in need an effective amount of a compound of formula **I'** as defined above or a pharmaceutical composition thereof.

In another embodiment the invention relates to a compound of formula **I'** or to a pharmaceutical composition thereof for the use as defined above wherein each R¹ is independently selected from -H, -Bn, -Bz, -CO-C₁-C₄ alkyl and -C₁-C₄ alkyl and wherein each R¹ is independently optionally substituted by one or more groups R², preferably wherein each R¹ is independently selected from -H, -Bn, -Bz, -CO-CH₃ (Ac) and -C₁-C₄ alky and wherein each R¹ is independently optionally substituted by one or more groups R²; more preferably wherein each R¹ is independently selected from - H, Bn, Bz, Ac and -C₁-C₄ alkyl; and still more preferably wherein each R¹ is independently selected from -H and Ac.

In another embodiment, the invention relates to a compound of formula **I'** or to a pharmaceutical composition thereof for the use as defined above wherein X is selected from -C(=Se)-NHY', -SO₂-NYY' or -SO₂-OY, and still more preferably wherein X is -C(=Se)-NHY' or -SO₂-NYY'.

In another embodiment the invention relates to a compound of formula **I'** or to a pharmaceutical composition thereof for the use as defined above wherein Y is -C₂-C₁₈ alkyl optionally substituted by one or more groups R⁴; and preferably wherein Y is - C₂-C₁₈ alkyl.

In another embodiment the invention relates to a compound of formula **I'** or to a pharmaceutical composition thereof for the use as defined above, wherein Y' is H or -C₂-C₁₈ alkyl optionally substituted by one or more groups R⁴, and preferably wherein Y' is H.

In another embodiment, the invention relates to a compound of formula **I'** or a pharmaceutical composition thereof for the use as defined above wherein X is selected from -C(=Se)-NHY', -SO₂-NYY' or -SO₂-OY, and still more preferably wherein X is -C(=Se)-NHY' or -SO₂-NYY';
Y is -C₂-C₁₈ alkyl optionally substituted by one or more groups R⁴ and preferably wherein Y is -C₂-C₁₈ alkyl; and
Y' is H or -C₂-C₁₈ alkyl optionally substituted by one or more groups R⁴, and preferably wherein Y' is H.

In another embodiment, the invention relates to the compound of formula **I'** or a pharmaceutical composition thereof for the use as defined above is selected from:
**1** 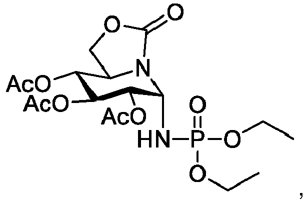
**2** 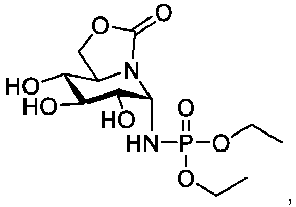
**3** 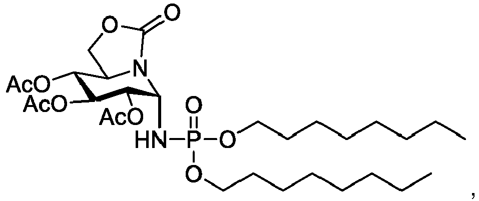
**4** 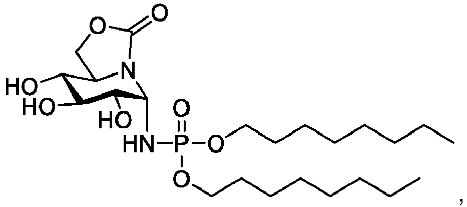
**5** 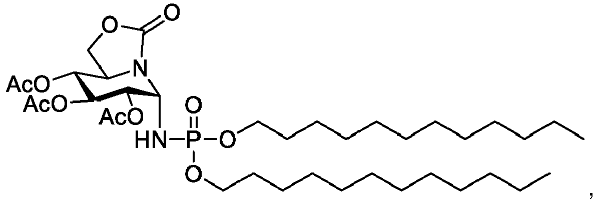
**6** 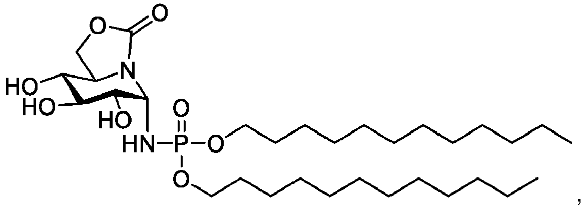
**7** 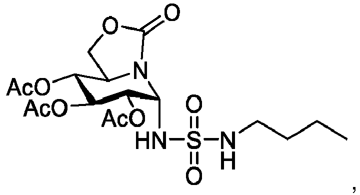
**8** 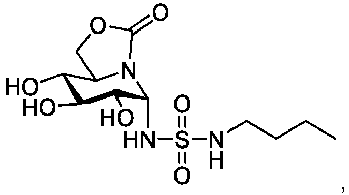
**9** 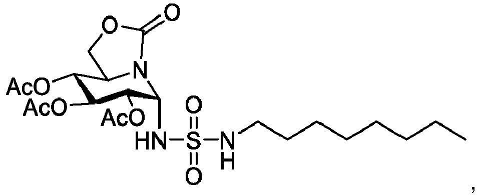
**10** 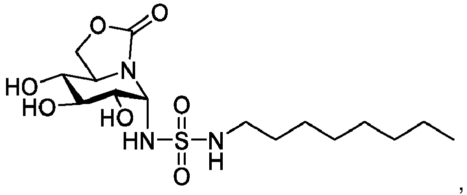
**11** 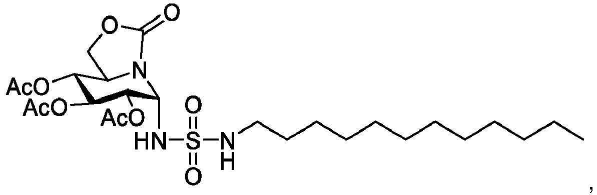
**12** 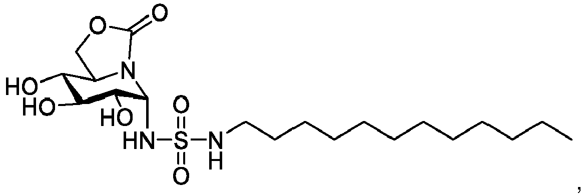
**13** 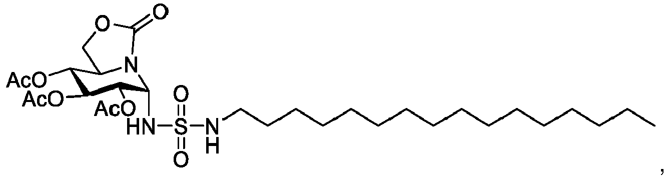
**14** 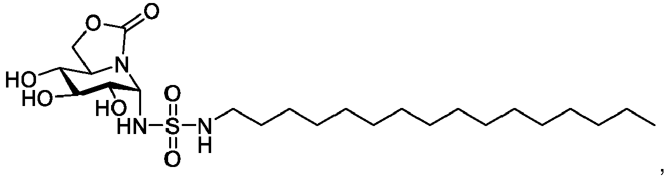
**15** 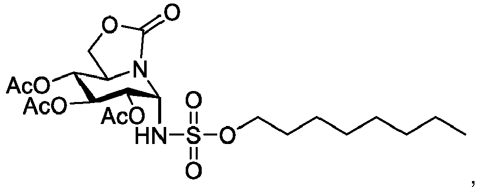
**16** 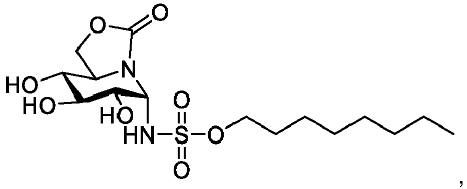
**17** 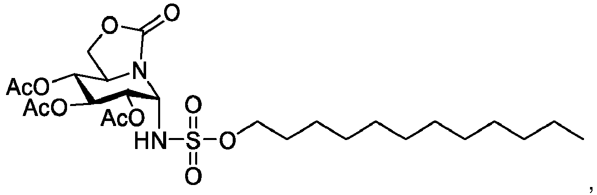
**18** 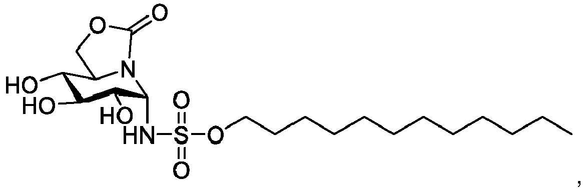
**19** 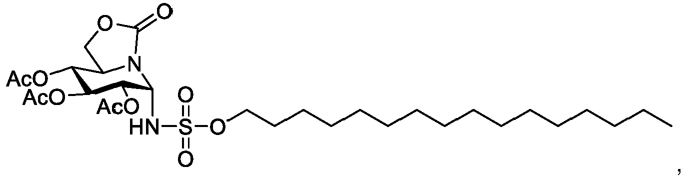
**20** 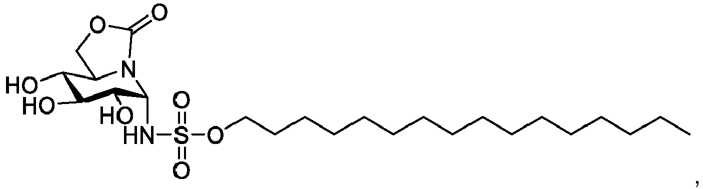
**21** 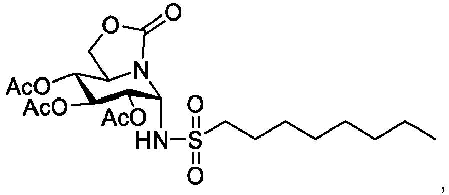
**22** 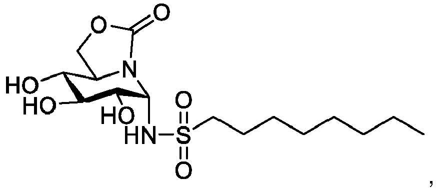
**23** 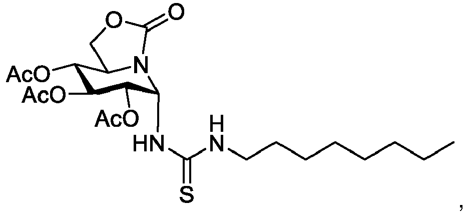
**24** 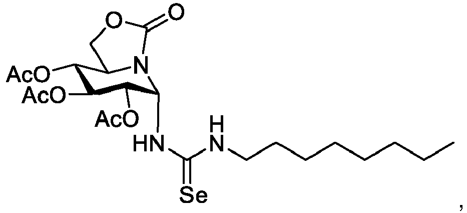
**25** 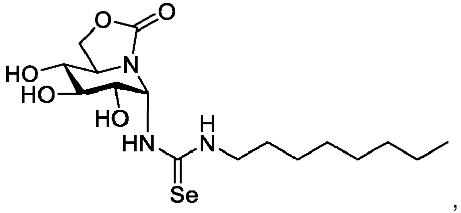
**26** 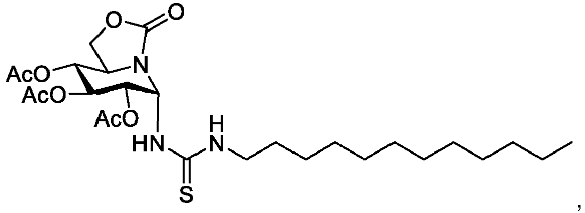
**27** 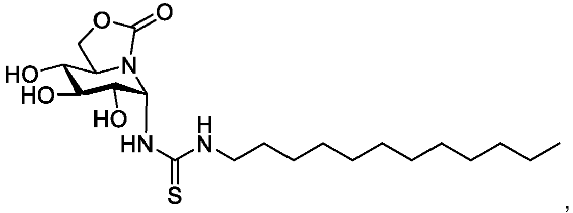
**28** 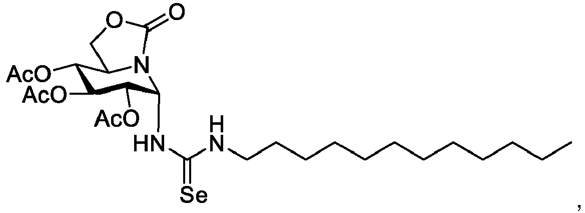
**29** 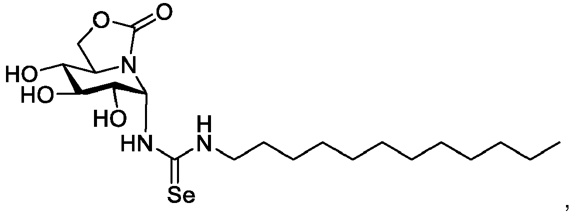
**30** 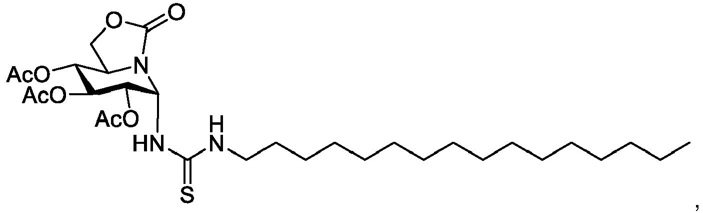
**31** 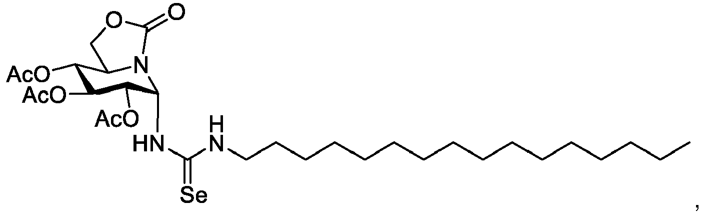
**32** 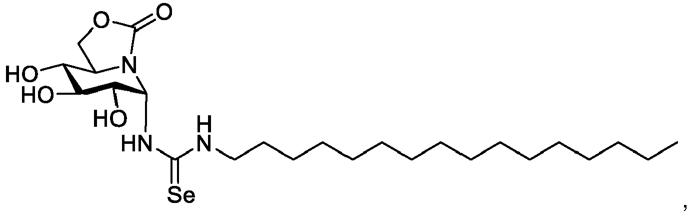
**33** 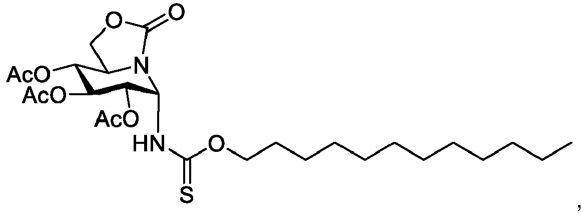 and
**34**

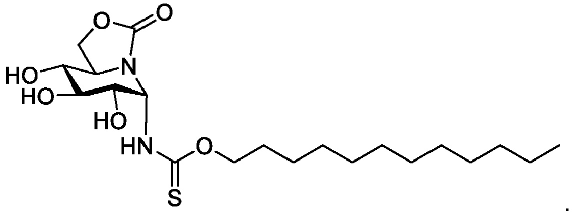

In another embodiment the invention relates to a compound of formula **I'** or a pharmaceutical composition thereof for the use defined above, wherein the immune disease is selected from acute inflammation, chronic disease allergy, cancer chemotherapy, infectious disease, the Metabolic Syndrome, non-alcoholic fatty liver disease (NAFLD), non-alcoholic steatohepatitis (NASH), immune mediated hepatitis, an autoimmune disease, graft rejection pathology, inflammatory bowel disease, atherosclerosis and airway hyperactivity, preferably wherein the immune disease is selected from acute inflammation, chronic disease allergy, infectious disease, immune mediated hepatitis, an autoimmune disease, inflammatory bowel disease, atherosclerosis and airway hyperactivity, and more preferably wherein the immune disease is selected from chronic disease allergy, an autoimmune disease and airway hyperactivity.

In another embodiment the invention relates to a compound of formula **I'** or a pharmaceutical composition thereof for the use defined above, wherein the airway hyperactivity is selected from asthma and allergic rhinitis.

The pharmaceutical composition of the invention may comprise as an active ingredient any one of: (a) one of the compounds of formula **I'**; or (b) a mixture of at least two compounds of formula **I'.** It should be noted that the therapeutic composition of the invention may optionally further comprise at least one of a pharmaceutically acceptable carrier, diluent, excipient or additive.

Throughout the present specification, by the term "treatment" is meant eliminating, reducing or ameliorating the cause or the effects of a disease. For purposes of this invention treatment includes, but is not limited to, alleviation, amelioration or elimination of one or more symptoms of the disease; diminishment of the extent of the disease; stabilized (i.e. not worsening) state of disease; delay or slowing of disease progression; amelioration or palliation of the disease state; and remission of the disease (whether partial or total).

As used herein, "prevention" refers to preventing the occurrence of a disease in a subject that is predisposed to or has risk factors but does not yet display symptoms of the disease. Prevention includes also preventing the recurrence of a disease in a subject that has previously suffered said disease.

Another aspect of the invention relates to a vaccine which comprises an effective amount of a compound of formula **I'** or a pharmaceutical salt thereof and a vaccine agent.

The therapeutic composition of the invention acts then as an immunologic adjuvant o as a modulator of a co-administered adjuvant and is capable of augmenting the immune response elicited by the vaccine agent by stimulating the immune system, which results in the subject responding to the vaccine more vigorously than without the compound while preserving a favorable safety profile. In other instances, the therapeutic composition acts as an immunodepleting agent, allowing preventing or decreasing unwanted immunological reactions to the vaccine in the treated subject.

In another embodiment, the invention relates to the vaccine defined above, wherein the compound of the invention is co-administered with an adjuvant selected form aluminum (Alum) based adjuvants comprising Aluminium Phosphate Gel and Aluminium Hydroxide Gel, or monophosphoryl lipid A (MPL^{®}).

In another embodiment the invention relates to the vaccine defined above, wherein the vaccine agent is selected from a killed microorganism, a live attenuated microorganism, a toxoid and a fragment of an inactivated or attenuated microorganism, a synthetic peptide or glycopeptide construct, a ribonucleic acid (RNA)-based vaccine or a deoxyribonucleic acid (DNA)-based vaccine.

In another embodiment the invention relates to the vaccine defined above, wherein the microorganism of the vaccine agent is a virus, a bacterium or a fungus.

In another embodiment the invention relates to the vaccine defined above, wherein the toxoid is a tetanus or a diphtheria toxoid.

In another embodiment the invention relates to the vaccine defined above, wherein the RNA is a messenger RNA (mRNA).

In another embodiment the invention relates to the vaccine defined above, wherein the DNA is a plasmid DNA (pDNA).

In another embodiment the RNA-based vaccine or the DNA-based vaccine comprises a pharmaceutically acceptable delivery system.

In another embodiment the delivery system is non-viral based or viral-based.

In another embodiment the non-viral based delivery system is selected from cationic lipids, cationic polymers, molecule-based systems and nanoparticle-based system. In another embodiment the viral based delivery system is selected from engineered adenovirus and engineered lentivirus systems.

Still further, another aspect of the invention relates to a method for the preparation of a medicament for the treatment of a pathologic disorder or condition in a subject in need thereof comprising the steps of: (A) providing an immunomodulatory comprising any one of: (a) at least one of the compounds of formula I'; (b) a mixture of at least two compounds of formula **I'**; and (B) admixing the immunomodulatory compound provided in step (A) with a pharmaceutically acceptable carrier.

Considering the above, this invention provides a novel glycolipid mimetic of formula **I** that is metabolically stable and can be selectively modified to optimize the TLR4 antagonist or agonist behavior. The present invention also aims to provide a method to prepare the said glycolipid mimetic with total α-anomeric stereoselectivity. Further on, the present invention provides a medicament such as an anti-inflammatory agent or a vaccine formulation containing a glycolipid mimetic of formula I'.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

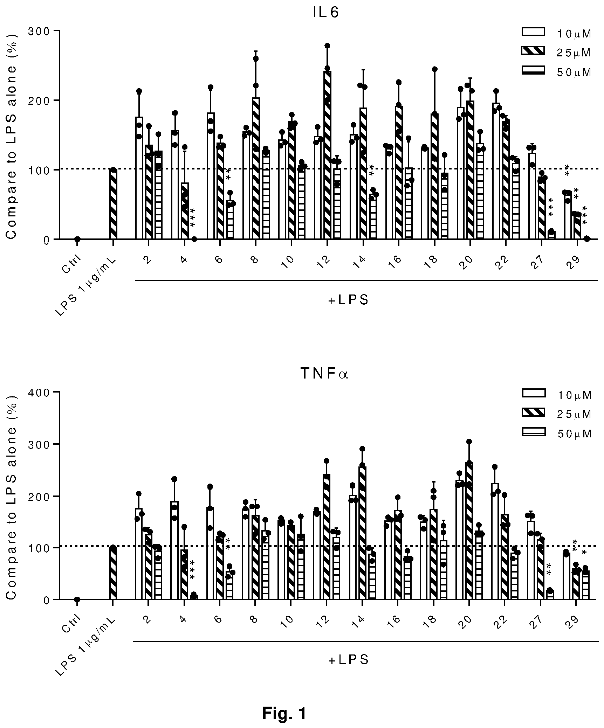

**Fig. 1****.** Shows suppression of LPS-induced inflammation (Splenocytes *in vitro).* Splenocytes were treated concurrently with the compounds of the invention and LPS to assess the suppressive function of compounds on LPS-induced inflammation, with several compounds showing prominent suppressive function.

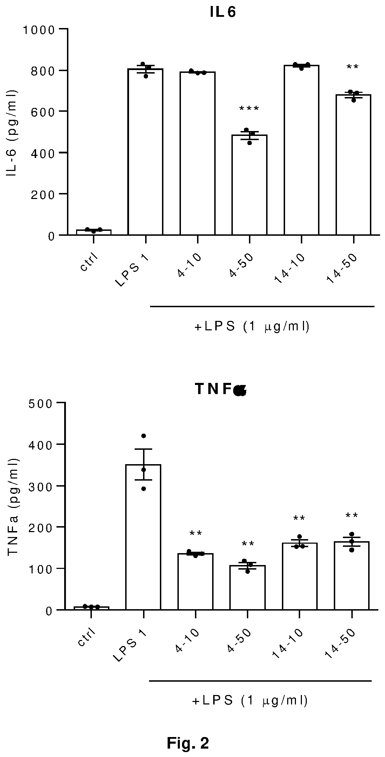

**Fig. 2****.** Shows suppression of LPS-induced inflammation in bone marrow-derived macrophages (BMDM *in vitro).* Compounds **4** and **14** were assessed for their capability to suppress LPS-induced inflammation in bone marrow-derived macrophages (BMDM), with both showing significant suppression of IL-6 at 50 µM and TNFα at 10 and 50 µM.

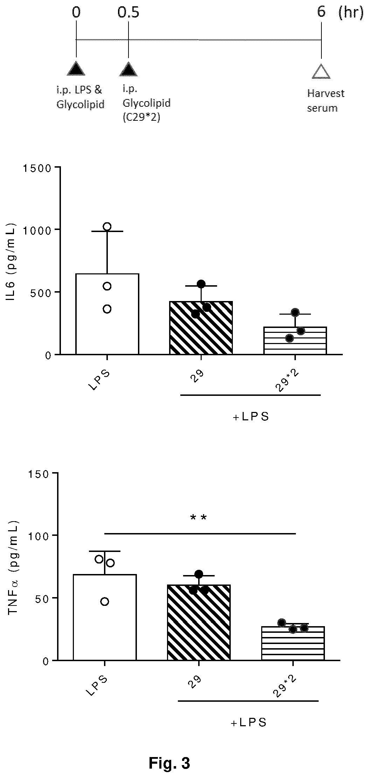

**Fig. 3****.** Shows suppression on LPS-induced inflammation *in vivo.* The *in vivo* capability of compound **29** in suppressing LPS-induced inflammation in B6 mice was assessed as indicated in the scheme. Compound **29** was shown to significantly suppressing LPS-induced serum TNFa when injected twice in a half hour time after LPS-treatment.

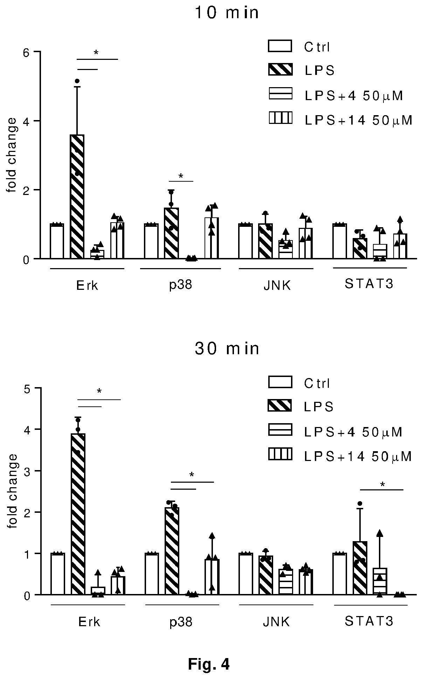

**Fig. 4****.** Shows ERK and p38 of the MAPK family and STAT3 are suppressed by the new compounds. Compounds **4** and **14,** the representative compounds of the invention, were examined in term of signal transduction to explore how the new compounds exert their suppressive effect, using the multiplex bead array. Comparing to LPS treatment alone, the phosphorylation of mitogen-activated protein kinases (MAPKs) ERK and p38, but not JNK, was significantly reduced by compounds **4** and **14** at 30 min post-treatment. A significant reduction of phosphorylation in STAT3 by compound **14** was also observed at 30 min post-treatment.

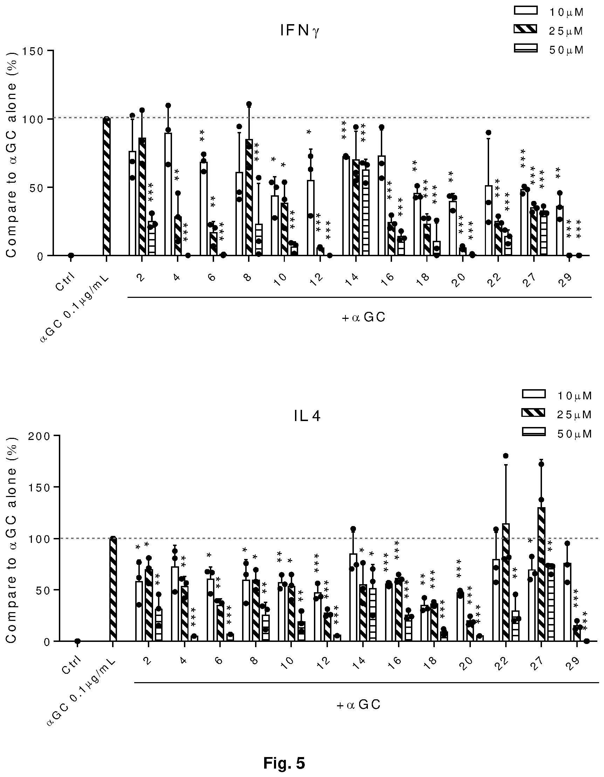

**Fig. 5****.** Shows suppression of αGalCer-induced inflammation (Splenocytes *in vitro).* Splenocytes were treated concurrently with the compounds of the invention and αGalCer to assess the suppressive function of compounds on αGaICer-induced inflammation, with several compounds showing prominent suppressive function.

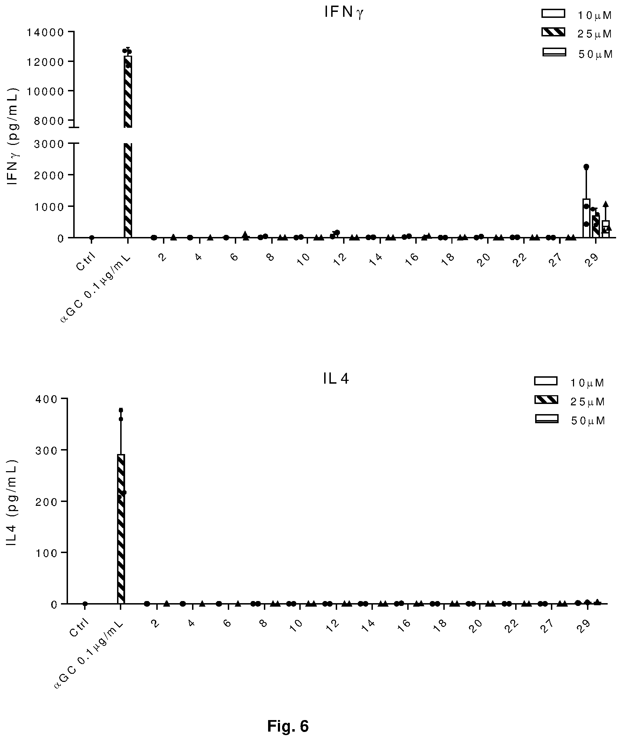

**Fig. 6****.** Shows immune-stimulating function of compounds (Splenocytes *in vitro).* Splenocytes were treated with the compounds of the invention alone to assess the immune-stimulating function of the compounds. Only compound **29** induced significant IFNγ secretion, and no compound could induce significant IL-4 secretion.

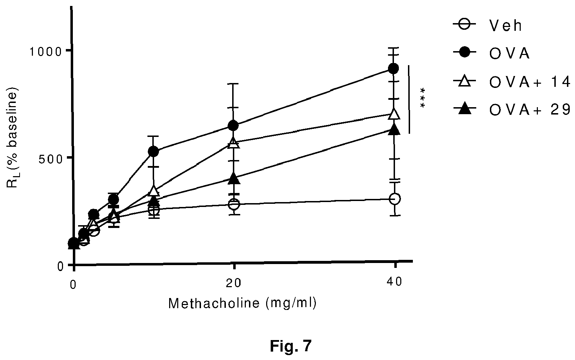

**Fig. 7****.** Shows suppression on ovalbumin (OVA)-induced airway hyperreactivity (AHR) *in vivo.* The capability of selected compounds **14** and **29** in suppressing Th2 inflammation *in vivo* was assessed using the OVA-induced AHR mouse model. Compound **29** was observed to suppress AHR significantly.

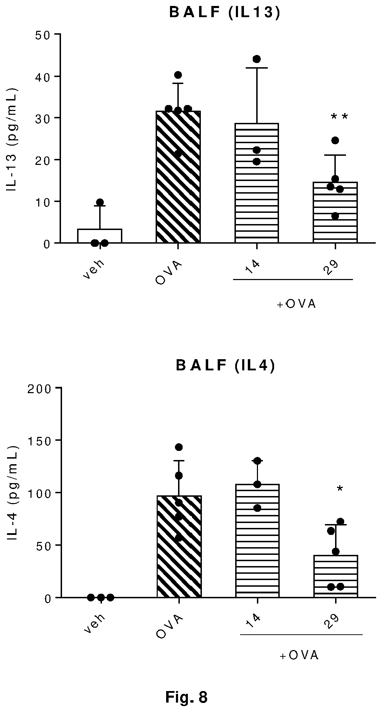

**Fig. 8****.** Shows suppression on OVA-induced airway inflammation *in vivo* (BALF). The bronchoalveolar lavage fluid (BALF) from mice in the OVA-induced AHR experiment was examined for their IL-13 and IL-4 levels. Compound **29** was shown to suppress both IL-13 and IL-4 levels significantly.

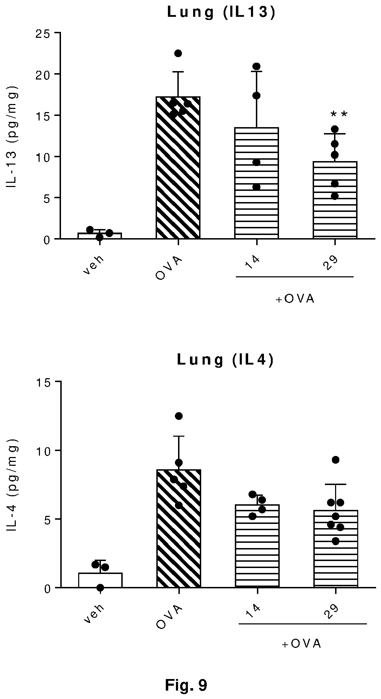

**Fig. 9****.** Shows suppression on OVA-induced airway inflammation *in vivo* (Lung protein). The lungs from mice in the OVA-induced AHR experiment were examined for their IL-13 and IL-4 protein levels. Only compound **29** showed significant suppression on IL-13, and no significant suppression of IL-4 was observed in compounds examined.

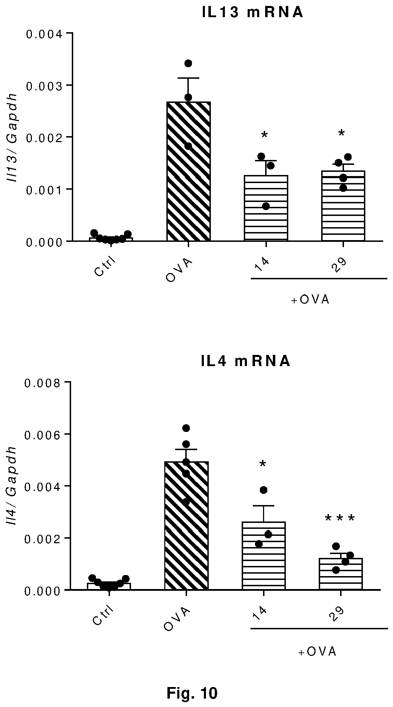

**Fig. 10****.** Shows suppression on OVA-induced airway inflammation *in vivo* (Lung mRNA). The lungs from mice in the OVA-induced AHR experiment were examined for their IL-13 and IL-4 mRNA levels. Both compounds **14** and **29** significantly suppressed IL-13 and IL-4 mRNA levels.

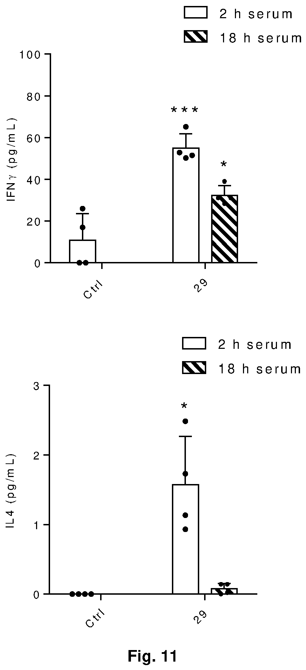

**Fig. 11****.** Shows the adjuvancy function of compounds *in vivo.* The adjuvancy capability of the selected compound **29** *in vivo* was assessed by measuring the serum level of IFNγ and IL-4 induced after injecting them into B6 mice. Compound **29** showed significant capability in inducing both IFNy and IL-4 2 hours after injection and still with a significant detectable level of IFNγ at 18 hours after injection.

### Examples

The following examples are intended to illustrate embodiments of the invention and are not intended to be construed in a limiting manner

### Methods

All reagents and solvents used in the preparations disclosed in the following examples were purchased from commercial sources and used without further purification, unless otherwise specified. Thin-layer chromatography (TLC) was carried out on aluminum sheets coated with Silica gel 60 F₂₅₄ Merck with visualization by UV light (λ 254 nm) and by charring with 10% ethanolic H₂SO₄, 0.1% ethanolic ninhydrin and heating at 100 °C. With preparative purposes, column chromatography was carried out on Silice 60 A.C.C. Chromagel (SDS 70-200 and 35-70 µm). Optical rotations were measured at 20 ± 2 °C in 1 cm tubes on a Jasco P-2000 polarimeter using a sodium lamp (λ 589 nm). Elemental analyses were carried out at the Instituto de Investigaciones Químicas (Sevilla, Spain) using an elemental analyser Leco CHNS-932 o Leco TruSpec CHN. NMR experiments were performed at 300 (75.5), and 500 (125.7, 202) MHz with Bruker 300 ADVANCE and 500 DRX. 1D TOCSY, 2D COSY, HMQC and HSQC experiments were used to assist on NMR assignments. The chemical shift values are given in ppm (part per million), using the solvent as internal standard, tetramethylsilane (for CDCl₃). The values of the coupling constant (J) are measured in Hz. Abbreviations to indicate the multiplicities of the signals are: s (singlet), bs (broad singlet), d (doublet), t (triplet), q (quartet) and m (multiplet). Mass spectra (High-resolution mass spectra) were carried out on a Bruker Daltonics Esquire6000^{™} (LTQ-Orbitrap XL ETD). The samples were introduced via solid probe heated from 30 to 280 °C. ESI as ionization source (Electrospray Ionization) was used to which methanol was used as solvent. The samples were introduced via direct injection using a Cole-Parmer syringe at a flow rate of 2 µl/min. Ions were scanned between 300 and 3000 Da with a scan speed of 13000 Da/s at unit resolution using resonance ejection at the multipole resonance of one-third of the radio frequency (Ω = 781.25 kHz).

### Materials

In exemplary disclosures, the following precursors used in the synthesis of specific compounds of the invention were accessed through known methods:
(1*R*)-1,2,3,4-Tetra-*O*-acetyl-5*N*,6*O*-oxomethylidenenojirimycin **35** was prepared as described in V. M. Diaz Perez, M. I. Garcia Moreno, C. Ortiz Mellet, J. Fuentes, J. C. Diaz Arribas, F. J. Cañada and J. M. Garcia Fernandez, J. Org. Chem., 2000, 65, 136-143.

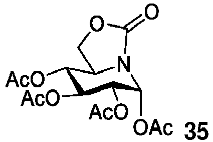

*N*-(Octyl)sulfamide was prepared as described in K. Suthagar, A. J. A. Watson, B. L. Wilkinson and A. J. Fairnbanks, Eur. J. Med. Chem., 2015, 102, 153-166.

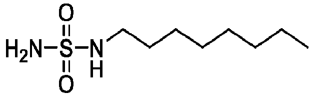

*N*-(Dodecyl)sulfamide was prepared as described in K. Suthagar, A. J. A. Watson, B. L. Wilkinson and A. J. Fairnbanks, Eur. J. Med. Chem., 2015, 102, 153-166.

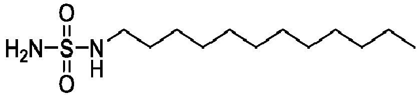

O-Octylsulfamate was prepared as described in S. Ahmed, K. James, C. P. Owen, C. K. Patela and L. Sampsonc, Bioorg. Med. Chem. Letters 2002, 12,1279-1282.

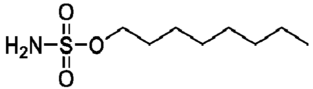

O-Dodecylsulfamate was prepared as described in J.-Y. Winum, D. Vullo, A. Casini, J.-L. Montero, A. Scozzafava, and C. T. Supuran, J. Med. Chem. 2003, 46, 5471-5477.

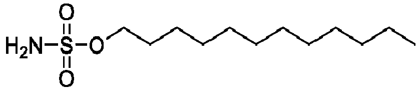

O-Hexadecylsulfamate was prepared as described in J.-Y. Winum, D. Vullo, A. Casini, J.-L. Montero, A. Scozzafava, and C. T. Supuran, J. Med. Chem. 2003, 46, 5471-5477.

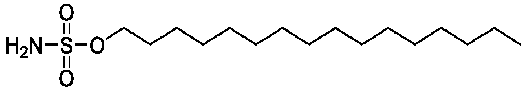

Octyl sulphonamide was prepared as described in D. Zhong, D. Wu, Y. Zhang, Z. Lu, M. Usman, W. Liu, X. Lu and W.-B. Liu, Org. Lett. 2019, 21, 5808-5812.

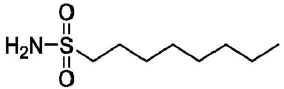

α-GalCer (αGC, KRN7000) was purchased from Funakoshi (Tokyo, Japan). RPMI 1640, DMEM, newborn calf serum (NBCS), and red blood cell (RBC) lysis buffer were purchased from Gibco (Waltham, MA, USA). Fetal bovine serum (FBS) was purchased from HyClone (Logan, UT, USA). Recombinant mouse IL-2 and GM-CSF were purchased from BioLegend (San Diego, CA, USA) and PeproTech (Rocky Hill, NJ, USA). Recombinant human IL-2 was purchased from eBioscience (San Diego, CA, USA). Ovalbumin (OVA) was purchased from Worthington Biochemicals (Freehold, NJ, USA).

### Mice

Eight- to ten-week-old C57BL/6 and BALB/c female mice were purchased from National Laboratory Animal Center (Taipei, Taiwan). All animals were housed under specific pathogen-free conditions.

### In vitro culture of mouse splenocytes and human peripheral blood mononuclear cells (PBMCs)

The whole spleen was surgically removed from a mouse and mechanically dissociated into a single cell suspension of splenocytes, which were then cultured in the complete RPMI 1640 medium (cRPMI). Human peripheral blood mononuclear cells (PBMCs) from healthy volunteers were collected from whole blood through gradient-centrifugation with Histopaque-1077 (Sigma-Aldrich).

### In vivo treatment

For ovalbumin (OVA) challenge, mice were sensitized intraperitoneally (i.p.) with 50 µg of OVA with 2% alhydrogel adjuvant (InvivoGen). Mice were challenged three times with 50 µg of OVA on day 7 post-sensitization, either daily or every other day. Mice were sacrificed the day after the last OVA challenge.

### Measurement of airway hyperreactivity (AHR)

Mice were anesthetized with 100 mg per kg body weight pentobarbital (Sigma-Aldrich), tracheotomised and mechanically ventilated via the FinePointe RC system (Buxco Research Systems, Wilmington, NC). Lung function was assessed by measuring lung resistance and dynamic compliance in response to increasing doses of aerosolized methacholine (1.25-40 mg/mL, Sigma-Aldrich).

### Collection and analysis of bronchoalveolar lavage fluid (BALF)

Upon exposure of the trachea, the lungs were lavaged twice with 1 mL of PBS supplemented with 2% fetal calf serum (FCS). BALF was pooled and BAL cells were pelleted by centrifugation and fixed onto cytospin slides. The slides were stained with Diff-Quik solution (Polysciences Inc) and BAL differential cell count was performed.

### Flow cytometry

Single cell suspensions were stained with fixable viability dye eFluor^{®} 780 (eBioscience) for 30 mins at 4°C and Fc receptors were blocked with anti-CD16/32 (BioLegend) blocking antibody prior to surface staining with monoclonal antibodies.

### Statistics

Data were analyzed using the GraphPad Prism (GraphPad Software, San Diego, CA, USA). Statistical analysis was determined using the Student's t-test and the two-way analysis of variance (ANOVA).

### EXAMPLE 1. Synthesis of compound 1.

Compound **1** was prepared by reaction of triethylphosphite (P(=O)(OEt)₃) with the glycosylazido derivative **36**. Compound **36** was prepared at its turn from compound **35** as depicted in Scheme 1, following the procedure described hereinafter.

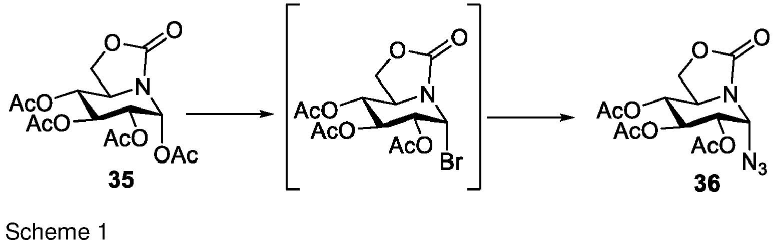

To a solution of **35** (100 mg, 0.267 mmol) in dry DCM (5 mL), cooled to 0 °C, a 33% HBr solution in AcOH (0.18 mL) was slowly added and the mixture was stirred for 5 min. Then the reaction was diluted with DCM (10 mL) and washed with aqueous NaHCO₃, dried (MgSO₄) and the solvent evaporated under reduced pressure. The bromo derivative thus formed was directly used in the next reaction without further purification. To a solution of the crude bromide in dry DMF (6.6 mL), NaN₃ (26 mg, 0.40 mmol) was added and the reaction mixture was stirred for 2 h under Ar atmosphere. The solvent was evaporated under reduced pressure and the residue was dissolved with EtOAc (20 mL), washed with H₂O (20 mL), dried (MgSO₄) and purified by colum chromatography (1:4 → 1:3 EtOAc-cyclohexane) to obtain **36**. Yield: 82 mg (86% yield over two steps). *R_{f}* 0.56 (2:1 EtOAc-cyclohexane). [α]_{D} -12.4 (c 1.0 in DCM). ¹H NMR (300 MHz, CDCl₃): δ = 5.25 (d, 1 H, *J*_{1,2} = 4.1 Hz H-1), 5.18 (dd, 1 H, *J*_{3,4} = 10.4 Hz, *J*_{4,5} = 6.6 Hz, H-4), 5.09-5.03 (m, 2 H, H-2, H-3), 4.47 (t, 1 H, *J*_{6a,6b} = 8.9 Hz, *J*_{5,6a} = 8.5 Hz, H-6a), 4.21 (t, 1 H, H-6b), 3.97 (m, 1 H, H-5), 2.13, 2.09, 2.06 (3 s, 9 H, CH₃CO). ¹³C NMR (75.5 MHz, CDCl₃): δ = 169.8-168.5 (CO ester), 156.5 (CO carbamate), 72.7-72.6 (C-3, C-4), 71.8 (C-2), 68.4 (C-5), 67.6 (C-6), 52.6 (C-1), 20.6 (CH₃). ESIMS: *m*/*z* = 379.1 [M + Na]⁺. Anal. calcd. for C₁₃H₁₆N₄O₈: C 43.82, H 4.53, N 15.73. Found: C 43.89, H 4.60, N 15.57.

In a subsequent step, to a solution of **36** (80 mg, 0.23 mmol) in dry DCM (1 mL), triethylphosphite (0.23 mmol, 1 eq) was added under Ar atmosphere. The mixture was stirred for 18 h. After complete formation of the phosphoramidate (TLC), H₂O (1 mL) was added to the reaction mixture and the solution was stirred for additional 5 h. The layers were separated, the organic layer was washed twice with brine, dried (MgSO₄) and the solvent evaporated under reduced pressure. The resulting residue was purified by column chromatography, eluent (4:1 acetone-toluene → MeOH), to give **1**. Yield: 86 mg (84%). *R_{f}*0.51 (1:1 acetone-toluene). [α]_{D} -3.73 (*c*1.0 in MeOH).¹H NMR (300 MHz, CDCl₃): δ 5.32 (t, 1 H, *J*_{2,3} = *J*_{3,4} = 9.3 Hz, H-3), 5.12 (m, 1 H, H-4, H-2), 4.79 (dd, 1 H, *J*_{1,2} = 9.6 Hz, *J*_{1,P} = 11.2 Hz, H-1), 4.36 (dd, 1 H, *J*_{6a,6b} = 9.5 Hz, *J*_{5,6a} = 6.9 Hz, H-6a), 4.19 (dd, 1 H, *J*_{5,6b} = 2.7 Hz H-6b), 4.09 (m, 4 H, CH₂O), 4.03 (m, 1 H, H-5), 2.08 (s, 6 H, CH₃CO), 2.06 (s, 6 H, CH₃CO), 2.00 (s, 3 H, CH₃CO), 1.3 (m, 6H, CH₃). ¹³C NMR (100.6 MHz, CDCl₃): δ 170.3, 169.9, 169.8 (CO ester), 156.7 (CO carbamate), 72.7 (C-3), 71.0 (C-2), 69.8 (C-4), 64.7 (C-1, C-6), 63.0 (CH₂O), 55.9 (C-5), 19.3 (*C*H₃CO), 19.1 (*C*H₃CO), 19.0 (*C*H₃CO), 15.1-14.9 (CH₃). ³¹P NMR (121.5 MHz, CDCl₃): δ = 6.49. ESIMS: *m*/*z* = 489.2 [M + Na]⁺. Anal. calcd. for C₁₇H₂₇N₂O₁₁P: C 43.78, H 5.84, N 6.01. Found: C 43.87, H 5.89, N 5.67.

### EXAMPLE 2. Synthesis of compound 2.

Compound **2** was obtained by treatment of a solution of **1** (65 mg, 0.139 mmol) in MeOH (3.5 mL) with 1 M NaOMe in MeOH (56 µL), followed by neutralization with Amberlite^{®} IR120 hydrogen form, filtration, evaporation and purification by colum chromatography (4:1 acetone-toluene → MeOH). Yield: 45 mg (95%). *R_{f}*0.50 (30:10:1 DCM-MeOH-H₂O). [α]_{D} -1.09 (c 1.0 in MeOH). ¹H NMR (500 MHz, CD₃OD): δ 4.36 (bd, 1 H, H-1), 4.31 (m, 2 H, H-2, H-4), 4.13 (m, 4 H, CH₂O), 3.58 (m, 1 H, H-5), 3.32 (m, 2 H, H-6a, H-6b), 3.24 (bdd, 1 H, H-3), 1.31 (bt, 6 H, CH₃).¹³C NMR (125.7 MHz, CD₃OD): δ 157.4 (CO carbamate), 76.8 (C-3), 73.1 (C-4), 71.6 (C-2), 67.1 (C-6), 64.9 (C-1), 62.7 (CH₂O), 58.3 (C-5), 15.0 (CH₃). ESIMS: *m*/*z* = 363.1 [M + Na]⁺. Anal. calcd. for C₁₁H₂₁N₂O₈P: C 38.83, H 6.22, N 8.23. Found: C 38.73, H 6.02, N 8.01.

### EXAMPLE 3. Synthesis of compound 3.

To a solution of **36** (80 mg, 0.23 mmol) in dry DCM (1 mL), trioctylphosphite (0.23 mmol, 1 eq) was added under Ar atmosphere. The mixture was stirred for 18 h. After complete formation of the phosphoramidate (TLC), H₂O (1 mL) was added to the reaction mixture and the solution was stirred for additional 5 h. The layers were separated, the organic layer was washed twice with brine, dried (MgSO₄) and the solvent evaporated under reduced pressure. The resulting residue was purified by column chromatography, eluent (1:3 → 1:1 → 4:1 EtOAc-cyclohexane). Yield: 100 mg (70%). *R_{f}*0.25 (1:1 EtOAc-cyclohexane). [α]_{D} -2.12 (c 1.0 in DCM). ¹H NMR (300 MHz, CDCl₃): δ 5.23 (t, 1 H, *J*_{2,3} = *J*_{3,4} = 9.5 Hz, H-3), 5.07 (t, 1 H, *J*_{4,5} = 9.5, H-4), 5.06 (t, 1 H, H-2), 4.73 (bdd, 1 H, *J*_{1,NH} = 11.5 Hz, *J*_{1,2} = 10.8 Hz, H-1), 4.54 (bdd, 1 H, *J*_{NH,P} = 9.9 Hz, NH), 4.28 (bdd, 1 H, H-6a), 4.15 (m, 1 H, H-6b), 3.9 (m, 4 H, CH₂O), 3.75 (m, 1 H, H-5), 2.07, 2.01 (2 s, 9 H, CH₃CO), 1.76-0.70 (m, 30 H, CH₂, CH₂C*H*₃). ¹³C NMR (75.5 MHz, CDCl₃): δ 169.8, 169.6, 169.5 (CO ester), 156.4 (CO carbamate), 72.4 (C-3), 70.4 (C-2), 69.7 (C-4), 67.4 (CH₂O), 65.6 (C-1), 64.5 (C-6), 56.4 (C-5), 46.6-10.5 (*C*H₃CO, CH₂, CH₂*C*H₃). ESIMS: *m*/*z=* 635.4 [M + H]⁺. Anal. calcd. for C₂₉H₅₁N₂O₁₁P: C 54.88, H 8.10, N 4.41. Found C 55.12, H 7.96, N 4.41.

### EXAMPLE 4. Synthesis of compound 4.

Compound **4** was obtained by treatment of a solution of **3** (100 mg, 0.157 mmol) in MeOH (4 mL) with 1 M NaOMe in MeOH (63 µL) followed by neutralization with Amberlite^{®} IR120 hydrogen form, filtration, evaporation and purification by colum chromatography (50:10:1 DCM-MeOH-H₂O). Yield: 68 mg (85%). *R_{f}* 0.70 (50:10:1 DCM-MeOH-H₂O). [α]_{D} -6.55 (c 1.0 in DCM). ¹H NMR (500 MHz, CDCl₃): δ 4.26 (bd, 1 H, H-1), 4.21 (bt, 2 H, H-2, H-4), 3.97 (m, 4 H, CH₂O), 3.43 (bt, 1 H, *J*_{4,5} = *J*_{5,6a} = 8.49 Hz, H-5), 3.31 (m, 2 H, H-6a, H-6b), 3.23 (t, 1 H, *J*_{2,3} = *J*_{3,4} = 9 Hz, H-3), 1.6-0.7 (m, 30 H, CH₂, CH₂C*H₃*). ¹³C NMR (125.7 MHz, CDCl₃): δ 157.5 (CO carbamate), 73.2 (C-3), 71.2 (C-6), 67.4 (C-2), 67.1 (C-4), 65.6 (CH₂O), 65.0 (C-1), 58.4 (C-5), 45.7-10.5 (CH₂, CH₂*C*H₃).³¹P NMR (121.5 MHz, CDCl₃): δ = 6.83. ESIMS: *m*/*z* = 531.3 [M + Na]⁺. Anal. calcd. for C₂₃H₄₅N₂O₈P: C 54.32, H 8.92, N 5.51. Found C 54.07, H 8.71, N 5.22.

### EXAMPLE 5. Synthesis of compound 5.

To a solution of **36** (80 mg, 0.23 mmol) in dry DCM (1 mL), tridodecylphosphite (0.23 mmol, 1 eq) was added under Ar atmosphere. The mixture was stirred for 18 h. After complete formation of the phosphoramidate (TLC), H₂O (1 mL) was added to the reaction mixture and the solution was stirred for additional 5 h. The layers were separated, the organic layer was washed twice with brine, dried (MgSO₄) and the solvent evaporated under reduced pressure. The resulting residue was purified by column chromatography, eluent (1:3 → 4:1 EtOAc-cyclohexane). Yield: 82 mg (79%). *R_{f}* 0.45 (2:1 EtOAc-Cyclohexane). [α]_{D} +8.01 (c 1.0 in DCM). ¹H NMR (300 MHz, CDCl₃): δ 5.24 (t, 1 H, *J*_{2,3} = *J*_{3,4} = 9.5 Hz, H-3), 5.07 (t, 1 H, *J*_{4,5} = 9.6, H-4), 5.06 (t, 1 H, H-2), 4.74 (bdd, 1 H, *J*_{1,NH} = 11.7 Hz, *J*_{1,2} = 10.8 Hz, H-1), 4.53 (bdd, 1 H, *J*_{NH,P} = 9.4 Hz, NH), 4.29 (dd, 1 H, *J*_{6a,6b} = 9.0 Hz, *J*_{5,6a} = 6.5 Hz, H-6a), 4.16 (bdd, 1 H, H-6b), 3.96 (m, 4 H, CH₂O), 3.75 (m, 1 H, H-5), 2.07, 2.02 (2 s, 9 H, CH₃CO), 1.65-0.7 (m, 50 H, CH₂, CH₂C*H₃*). ¹³C NMR (75.5 MHz, CDCl₃): δ 169.8, 169.7, 169.5 (CO ester), 156.4 (CO carbamate), 72.4 (C-3), 70.4 (C-2), 69.7 (C-4), 67.0 (CH₂O), 65.6 (C-1), 64.5 (C-6), 56.5 (C-5), 31.9-34.0 (*C*H₃CO, CH₂, CH₂*C*H₃). ³¹P NMR (121.5 MHz, CDCl₃): δ = 5.99. ESIMS: *m*/*z* = 769.4 [M + Na]⁺. Anal. calcd. for C₃₇H₆₇N₂O₁₁P: C 59.50, H 9.04, N 3.75. Found: C 59.39, H 9.11, N 3.62.

### EXAMPLE 6. Synthesis of compound 6.

Compound **6** was obtained by treatment of a solution of **5** (80 mg, 0.107 mmol) in MeOH (2.7 mL) with 1 M NaOMe in MeOH (43 µL) followed by neutralization with Amberlite^{®} IR120 hydrogen form, filtration, evaporation and purification by column chromatography (70:10:1 DCM-MeOH-H₂O). Yield: 65 mg (97%). *R_{f}* 0.70 (70:10:1 DCM-MeOH-H₂O). [α]_{D} +10.70 (c 1.0 in DCM). ¹H NMR (500 MHz, CDCl₃): δ 4.29 (bdd, 1 H, H-1), 4.23 (m, 2 H, H-2, H-4), 3.98 (m, 4 H, CH₂O), 3.43 (bt, 1 H, H-5), 3.33 (m, 2 H, H-6a, H-6b), 3.24 (t, 1 H, *J*_{2,3} = *J*_{3,4} = 9.2 Hz, H-3), 1.62-0.84 (m, 50 H, CH₂, CH₂C*H₃*). ¹³C NMR (125.7 MHz, CDCl₃): δ 157.6 (CO carbamate), 73.0 (C-3), 71.1 (C-6), 67.3 (C-2), 67.2 (C-4), 67.1 (CH₂O), 65.1 (C-1), 58.3 (C-5), 31.8-13.9 (CH₂, CH₂*C*H₃). ³¹P NMR (121.5 MHz, CDCl₃): δ = 7.07. ESIMS: *m*/*z* = 643.4 [M + Na]⁺. Anal. calcd. for C₃₁H₆₁N₂O₈P: C 59.98, H 9.90, N 4.51. Found: C 59.71, H 9.80, N 4.19.

### EXAMPLE 7. Synthesis of compound 7.

To a solution of **35** (50 mg, 0.133 mmol) in dry DCM (1 mL), commercial N-(butyl)sulfamide (0.159 mmol) and BF₃·OEt₂ (33 µL, 0.266 mmol) were added under Ar atmosphere at 0 °C. The mixture was stirred for 16 h. After complete formation of the product (TLC), Et₃N (0.15 mL) was added and the mixture was stirred for 10 min and concentrated. The resulting residue was purified by column chromatography, eluent (1:2 → 1:1 EtOAc-cyclohexane), to afford **7.** Yield: 48 mg (77%). *R_{f}* 0.60 (2:1 EtOAc-cyclohexane). [α]_{D} +46.10 (c 1.0 in DCM). ¹H NMR (300 MHz, CDCl₃): δ 5.45 (t, 1 H, *J*_{2,3} = *J*_{3,4} = 9.5 Hz, H-3), 5.45 (bdd, 1 H, *J*_{1,2} = 5.0 Hz, *J*_{1,NH} = 3.0 Hz, H-1), 5.39 (d, 1 H, NH), 5.16 (t, 1 H, *J*_{NH,CH2} = 5.8 Hz, N*H*-CH₂), 5.06 (dd, 1 H, H-2), 4.94 (t, 1 H, *J*_{4,5} = 9.4 Hz, H-4), 4.46 (bdd, 1 H, *J*_{6a,6b} = *J*_{5,6a} = 8.8 Hz, H-6a), 4.27 (bdd, 1 H, H-6b), 4.15 (m, 1 H, H-5), 2.96 (m, 2 H, CH₂N), 2.09, 2.04, 2.02 (3 s, 9 H, CH₃CO), 1.52 (m, 2 H, CH₂), 1.35 (m, 2 H, CH₂), 0.89 (t, 3 H, CH₃). ¹³C NMR (75.5 MHz, CDCl₃): δ 169.1, 168.8, 168.1 (CO ester), 155.1 (CO carbamate), 71.4 (C-4), 68.1 (C-2), 68.1 (C-3), 66.1 (C-6), 58.7 (C-1), 50.5 (C-5), 42.0 (CH₂N), 19.5 (CH₂, CH₃CO), 18.7 (CH₂), 12.5 (CH₃). ESIMS: *m*/*z* = 488.1 [M + Na]⁺. Anal. calcd. for C₁₇H₂₇N₃O₁₀S: C 43.87, H 5.85, N 9.03, S 6.89. Found: C 43.81, H 5.77, N 8.89, S 6.61.

### EXAMPLE 8. Synthesis of compound 8.

Compound **8** was obtained by treatment of a solution of **7** (66 mg, 0.141 mmol) in MeOH (3.5 mL) with 1 M NaOMe in MeOH (56 µL), followed by neutralization with Amberlite^{®} IR120 hydrogen form, filtration, evaporation and freeze drying. Yield: 48 mg (quantitative). *R_{f}* 0.30 (80:10:1 DCM-MeOH-H₂O). [α]_{D} +32.53 (*c* 1.0 in MeOH). ¹H NMR (300 MHz, CD₃OD): δ 5.19 (d, 1 H, *J*_{1,2} = 4.7 Hz, H-1), 4.51 (t, 1H, *J*_{6a,6b} = 8.7 Hz, H-6a), 4.29 (dd, 1 H, *J*_{5,6a} = 5.2 Hz, H-6b), 3.83 (m, 1 H, H-5), 3.56 (m, 2 H, H-2, H-3), 3.32 (m, 1 H, H-4), 3.02 (m, 2 H, CH₂N), 1.55 (m, 2 H, CH₂), 1.40 (m, 2 H, CH₂), 0.96 (t, 3 H, CH₃). ¹³C NMR (75.5 MHz, CD₃OD): δ 156.9 (CO carbamate), 73.8 (C-4), 73.09 (C-2), 70.0 (C-3), 66.5 (C-6), 62.9 (C-1), 53.2 (C-5), 42.2 (CH₂N), 31.3 (CH₂), 19.5 (CH₂), 12.6 (CH₃). ESIMS: *m*/*z* = 362.13 [M + Na]⁺. Anal. calcd. for C₁₁H₂₁N₃O₇S: C 38.93, H 6.24, N 12.38, S 9.45. Found: C 38.76, H 6.04, N 12.11, S 9.18. **EXAMPLE 9.** Synthesis of compound **9**.

To a solution of **35** (50 mg, 0.133 mmol) in dry DCM (1 mL), *N*-(octyl)sulfamide (0.159 mmol) and BF₃·OEt₂ (33 µL, 0.266 mmol) were added under Ar atmosphere at 0 ºC. The mixture was stirred for 16 h. After complete formation of the product (TLC), Et₃N (0.15 mL) was added and the mixture was stirred for 10 min and concentrated. The resulting residue was purified by column chromatography, eluent (1:3 → 1:2 → 1:1 EtOAc-cyclohexane). Yield: 48 mg (70%). *R_{f}* 0.70 (2:1 EtOAc-cyclohexane). [α]_{D} +54.92 (c 1.0 in DCM). ¹H NMR (300 MHz, CDCl₃): δ 5.49 (t, 1 H, *J*_{2,3} = *J*_{3,4} = 9.5 Hz, H-3), 5.49 (bdd, 1 H, *J*_{1,2} = 5.0 Hz, *J*_{1,NH} = 2.7 Hz, H-1), 5.41 (d, 1 H, NH), 5.19 (t, 1 H, *J*_{NH,CH2}= 5.8 Hz, N*H*-CH₂), 5.10 (dd, 1 H, H-2), 4.98 (t, 1 H, *J*_{4,5} = 9.5 Hz, H-4), 4.50 (t, 1 H, *J*_{6a,6b} = *J*_{5,6a} = 8.5 Hz, H-6a), 4.30 (t, 1 H, H-6b), 4.18 (m, 1 H, H-5), 2.98 (m, 2 H, CH₂N), 2.13, 2.08, 2.06 (3 s, 9 H, CH₃CO), 1.73-0.8 (m, 15 H, CH₂, CH₂C*H₃*). ¹³C NMR (75.5 MHz, CDCl₃): δ 170.1, 169.7, 169.0 (CO ester), 156.2 (CO carbamate), 72.5 (C-4), 69.2 (C-2), 69.1 (C-3), 67.2 (C-6), 59.7 (C-1), 51.5 (C-5), 43.3 (CH₂N), 31.7-14.0 (*C*H₃CO, CH₂, CH₂*C*H₃). ESIMS: *m*/*z* = 544.2 [M + Na]⁺. Anal. calcd. for C₂₁H₃₅N₃O₁₀S: C 48.36, H 6.76, N 8.06, S 6.15. Found: C 48.48, H 6.85, N 7.85, S 5.92.

### EXAMPLE 10. Synthesis of compound 10.

Compound **10** was obtained by treatment of a solution of **9** (40 mg, 0.076 mmol) in MeOH (1.9 mL) with 1 M NaOMe in MeOH (30 µL), followed by neutralization with Amberlite^{®} IR120 hydrogen form, filtration, evaporation and freeze drying. Yield: 30 mg (quantitative). *R_{f}* 0.40 (80:10:1 DCM-MeOH-H₂O). [α]_{D} +33.97 (c 1.0 in acetone). ¹H NMR (300 MHz, CD₃OD): δ 5.19 (d, 1 H, *J*_{1,2}= 4.7 Hz, H-1), 4.51 (t, 1H, *J*_{6a,6b} = 8.7 Hz, H-6a), 4.29 (dd, 1 H, *J*_{5,6b} = 5.2 Hz, H-6b), 3.83 (m, 1 H, H-5), 3.57 (m, 2 H, H-2, H-3), 3.33 (m, 1 H, H-4), 3.01 (m, 2 H, CH₂N), 1.57-0.91 (m, 15 H, CH₂, CH₂C*H₃*). ¹³C NMR (75.5 MHz, CDCl₃): δ 156.9 (CO carbamate), 73.8 (C-4), 73.0 (C-2), 70.0 (C-3), 66.5 (C-6), 62.9 (C-1), 53.2 (C-5), 42.2 (CH₂N), 31.5-13.0 (CH₂, CH₂*C*H₃). ESIMS: *m*/*z* = 418.21 [M + Na]⁺. Anal. calcd. for C₁₅H₂₉N₃O₇S: C 45.56, H 7.39, N 10.63, S 8.11. Found: C 45.39, H 7.22, N 10.36, S 7.84.

### EXAMPLE 11. Synthesis of compound 11.

To a solution of **35** (50 mg, 0.133 mmol) in dry DCM (1 mL), *N*-(dodecyl)sulfamide (0.159 mmol) and BF₃·OEt₂ (33 µL, 0.266 mmol) were added under Ar atmosphere at 0 ºC. The mixture was stirred for 16 h. After complete formation of the product (TLC), Et₃N (0.15 mL) was added and the mixture was stirred for 10 min and concentrated. The resulting residue was purified by column chromatography, eluent (1:2 EtOAc-cyclohexane). Yield: 60 mg (78%). *R_{f}* 0.43 (1:1 EtOAc-cyclohexane). [α]_{D} +30.83 (c 1.0 in DCM). ¹H NMR (300 MHz, CDCl₃): δ 5.48 (t, 1 H, *J*_{2,3} = *J*_{3,4} = 9.5 Hz, H-3), 5.49 (bdd, 1 H, *J*_{1,2} = 5.0 Hz, *J*_{1,NH} = 2.9 Hz, H-1), 5.28 (d, 1 H, NH), 5.16 (t, 1 H, *J*_{NH,CH2} = 5.8 Hz, N*H*-CH₂), 5.10 (dd, 1 H, H-2), 4.97 (t, 1 H, *J*_{4,5} = 9.4 Hz, H-4), 4.50 (t, 1 H, *J*_{6a,6b} = *J*_{5,6a} = 8.6 Hz, H-6a), 4.30 (t, 1 H, H-6b), 4.18 (m, 1 H, H-5), 2.98 (m, 2 H, CH₂N), 2.13, 2.08, 2.06 (3 s, 9 H, CH₃CO), 1.66-0.89 (m, 25 H, CH₂, CH₂C*H₃*). ¹³C NMR (75.5 MHz, CDCl₃): δ 170.0, 169.7, 169.0 (CO ester), 156.2 (CO carbamate), 72.5 (C-4), 69.2 (C-2), 69.1 (C-3), 67.3 (C-6), 59.7 (C-1), 51.5 (C-5), 43.4 (CH₂N), 31.9-14.1 (*C*H₃CO, CH₂, CH₂*C*H₃). ESIMS: *m*/*z* = 600.3 [M + Na]⁺. Anal. calcd. for C₂₅H₄₃N₃O₁₀S: C 51.98, H 7.50, N 7.27, S 5.55. Found: C 52.20, H 7.63, N 7.33, S 5.32.

### EXAMPLE 12. Synthesis of compound 12.

Compound **12** was obtained by treatment of a solution of **11** (40 mg, 0.103 mmol) in MeOH (2.6 mL) with 1 M NaOMe in MeOH (42 µL), followed by neutralization with Amberlite^{®} IR120 hydrogen form, filtration, evaporation and freeze drying. Yield: 47 mg (quantitative). *R_{f}* 0.50 (80:10:1 DCM-MeOH-H₂O). [α]_{D} +20.83 (c 1.0 in acetone). ¹H NMR (300 MHz, 1:5 CDCl₃-CD₃OD): δ 5.15 (d, 1 H, *J*_{1,2} = 4.7 Hz, H-1), 4.48 (t, 1 H, *J*_{6a,6b} = 8.7 Hz, H-6a), 4.25 (dd, 1 H, *J*_{5,6b} = 5.4 Hz, H-6b), 3.81 (m, 1 H, H-5), 3.53 (m, 2 H, H-2, H-3), 3.29 (m, 1 H, H-4), 2.90 (m, 2 H, CH₂N), 1.54-0.86 (m, 25 H, CH₂, CH₂C*H₃*). ¹³C NMR (75.5 MHz, 1:5 CDCl₃-CD₃OD): δ 156.7 (CO carbamate), 73.6 (C-4), 72.8 (C-2), 69.6 (C-3), 66.3 (C-6), 62.2 (C-1), 52.8 (C-5), 42.3 (CH₂N), 31.4-12.9 (*C*H₃CO, CH₂, CH₂*C*H₃). ESIMS: *m*/*z* = 474.24 [M + Na]⁺. Anal. calcd. for C₁₉H₃₇N₃O₇S: C 50.54, H 8.26, N 9.31, S 7.10. Found: C 50.26, H 7.97, N 9.00, S 6.84.

### EXAMPLE 13. Synthesis of compound 13.

Compound **13** was obtained by conjugation of **35** and N-hexadecylsulfamide **38**. Precursor **37** was prepared from chlorosulfonyl isocyanate and hexadecylamine according to Scheme 2, through the procedure described hereinafter.

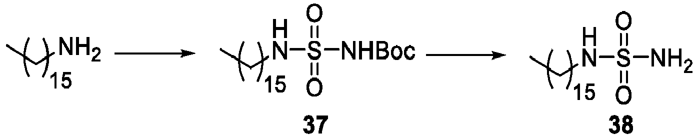

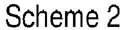

To a solution of commercial chlorosulfonyl isocyanate (4 mmol) in dry DCM (1.5 mL), ^{t}BuOH (6 mmol, 568 µL) was added slowly, under Ar atmosphere, at 0 ºC. The mixture was stirred 30 min at 0 °C. Hexadecylamine (4 mmol, 1eq) and dry Et₃N (1.5 eq) were dissolved in dry DCM (1.5 mL) and then added to the previous solution. The reaction mixture was stirred for 16 h at rt. After complete formation of the product, the solvent was evaporated under reduced pressure and the residue was dissolved with DCM, washed with aqueous NaHCO₃(3 × 20 mL), brine (3 × 20 mL) and dried (MgSO₄). The resulting residue was purified by column chromatography using DCM to afford the Boc-protected sulfamide derivative **37**. Yield: 117 mg (15%). *R_{f}* 0.2 (DCM). [α]_{D} -2.07 (c 1.0 in DCM).¹H NMR (300 MHz, CDCl₃): δ 5.31 (s, 1 H, N*H*CH₂), 3.07 (t, 2 H, *J*_{H,H} = 7.0 Hz, CH₂N), 1.72-1.27 (m, 28 H, CH₂), 0.89 (t, 3 H, CH₂C*H₃*). ¹³C NMR (75.5 MHz, CDCl₃): δ 150.2 (CO), 83.7 (*C*Me₃), 43.9-14.1 (CH₂, CH₂*C*H₃, C*Me₃*). ESIMS: *m*/*z* = 443.3 [M + Na]⁺. Anal. calcd. for C₂₁H₄₄N₂O₄S: C 59.96, H 10.54, N 6.66, S 7.62. Found: C 60.19, H 10.71, N 6.48, S 7.45.

Compound **38** was obtained by treatment of **37** (60 mg, 0.142 mmol) with DCM-TFA (1:8, 2 mL) during 5 h, followed by purification by colum chromatography (60:10:1 DCM-MeOH-H₂O). Yield: 45 mg (quantitative). *R_{f}* 0.60 (50:10:1 DCM-MeOH-H₂O). [α]_{D} -9.20 (c 1.0 in DCM). ¹H NMR (300 MHz, DMSO-d₆): δ 5.41 (s, 1 H, NH₂), 3.31 (s, 1 H, N*H*CH₂), 2.82 (m, 2 H, CH₂N) 1.44-0.85 (m, 33 H, CH₂, CH₂C*H₃*). ¹³C NMR (75.5 MHz, DMSO-d₆): δ 43.0 (CH₂N), 31.7-14.4 (CH₂, CH₂*C*H₃). ESIMS: m/*z* = 319.1 [M - H]⁻. Anal. calcd. for C₁₆H₃₆N₂O₂S: C 59.95, H 11.32, N 8.74, S 10.00. Found: C 59.82, H 11.26, N 8.57, S 9.77

In a subsequent step, to a solution of **35** (50 mg, 0.133 mmol) in dry DCM (1 mL), *N-*(hexadecyl)sulfamide **38** (0.159 mmol) and BF₃·OEt₂(33 µL, 0.266 mmol) were added under Ar atmosphere at 0 °C. The mixture was stirred for 16 h. After complete formation of the product (TLC), Et₃N (0.15 mL) was added, and the mixture was stirred for 10 min and concentrated. The resulting residue was purified by column chromatography, eluent (1:2 EtOAc-cyclohexane), to afford **13**. Yield: 62 mg (75%). *R_{f}* 0.78 (1:1 EtOAc-cyclohexane). [α]_{D} +36.55 (c 1.0 in DCM). ¹H NMR (300 MHz, CDCl₃): δ 5.44 (t, 1 H, *J*_{2,3} = *J*_{3,4} = 9.5 Hz, H-3), 5.43 (bdd, 1 H, *J*_{1,2} = 5.0 Hz, *J*_{1, NH} = 2.9 Hz, H-1), 5.25 (d, 1 H, NH), 5.12 (t, 1 H, *J*_{NH,CH2} = 5.9 Hz, N*H*-CH₂), 5.06 (dd, 1 H, H-2), 4.93 (t, 1 H, *J*_{4,5} = 9.4 Hz, H-4), 4.47 (t, 1 H, *J*_{6a,6b} = *J*_{5,6a} = 8.6 Hz, H-6a), 4.26 (t,

1 H, H-6b), 4.14 (m, 1 H, H-5), 2.94 (m, 2 H, CH₂N), 2.09, 2.04, 2.02 (3 s, 9 H, CH₃CO), 1.53-0.85 (m, 33 H, CH₂, CH₂C*H₃*). ¹³C NMR (75.5 MHz, CDCl₃): δ 170.1, 169.7, 169.0 (CO ester), 156.2 (CO carbamate), 72.5 (C-4), 69.2 (C-2), 69.1 (C-3), 67.2 (C-6), 59.7 (C-1), 51.5 (C-5), 43.4 (CH₂N), 31.9-14.1 (*C*H₃CO, CH₂, CH₂*C*H₃). ESIMS: *m*/*z=* 656.4 [M + Na]⁺. Anal. calcd. for C₂₉H₅₁ N₃O₁₀S: C 54.96, H 8.11, N 6.63, S 5.06. Found: C 55.19, H 8.35, N 6.54, S 4.80.

### EXAMPLE 14. Synthesis of compound 14.

Compound **14** was obtained by treatment of a solution of **13** (77 mg, 0.122 mmol) in MeOH (3 mL) with 1 M NaOMe in MeOH (42 µL), followed by neutralization with Amberlite^{®} IR120 hydrogen form, filtration, evaporation and freeze drying. Yield: 62 mg (quantitative). *R_{f}* 0.6 (80:10:1 DCM-MeOH-H₂O). [α]_{D} +24.15 (c 1.0 in acetone). ¹H NMR (300 MHz, 1:1 CDCl₃-CD₃OD): δ 5.07 (s, 1 H, NH), 4.86 (d, 1 H, *J*_{1,2} = 4.8 Hz, H-1), 4.23 (t, 1 H, *J*_{6a,6b} = 8.7 Hz, H-6a), 3.97 (dd, 1 H, *J*_{56b} = 5.8 Hz, H-6b), 3.57 (m, 1 H, H-5), 3.32 (m, 2 H, H-2, H-3), 3.05 (t, 1 H, H-4), 2.71 (m, 2 H, CH₂N) 1.28-0.59 (m, 33 H, CH₂, CH₂C*H₃*).¹³C NMR (75.5 MHz, 1:1 CDCl₃-CD₃OD): δ 156.3 (CO carbamate), 73.2 (C-4), 72.5 (C-2), 69.1 (C-3), 66.1 (C-6), 61.8 (C-1), 52.3 (C-5), 42.0 (CH₂N), 31.0-12.8 (*C*H₃CO, CH₂, CH₂*C*H₃). ESIMS: *m*/*z* = 530.32 [M + Na]⁺. Anal. calcd. for C₂₃H₄₅N₃O₇S: C 54.41, H 8.93, N 8.28, S 6.31. Found: C 54.53, H 8.99, N 8.02, S 5.98.

### EXAMPLE 15. Synthesis of compound 15.

To a solution of **35** (100 mg, 0.267 mmol) in dry DCM (12 mL), *O*-octylsulfamate (0.320 mmol) and BF₃·OEt₂ (66 µL, 0.534 mmol) were added under Ar atmosphere at 0 °C. The mixture was stirred for 16 h. After complete formation of the product (TLC), Et₃N (0.3 mL) was added, and the mixture was stirred for 10 min and concentrated. The resulting residue was purified by column chromatography, eluent (1:2 → 1:1 EtOAc-cyclohexane), to afford **15**. Yield: 80 mg (57%). *R_{f}* 0.40 (1:1 EtOAc-cyclohexane). [α]_{D} +33.76 (c 1.0 in DCM). ¹H NMR (300 MHz, CDCl₃): δ 5.75 (bs, 1 H, NH), 5.63 (d, 1 H, *J*_{1,2} = 5.0 Hz, H-1), 5.46 (t, 1 H, *J*_{2,3} = *J*_{3,4} = 9.9 Hz, H-3), 5.13 (dd, 1 H, H-2), 5.02 (t, 1 H, *J*_{4,5} = 9.5 Hz, H-4), 4.47 (t, 1 H, *J*_{6a,6b} = *J*_{5,6a} = 8.5 Hz, H-6a), 4.30 (bdd, 1 H, *J*_{5,6b} = 6.2 Hz, H-6b), 4.23 (m, 2 H, CH₂O) 4.13 (m, 1 H, H-5), 2.13, 2.08, 2.06 (3 s, 9 H, CH₃CO), 1.75-0.89 (m, 15 H, CH₂, CH₂C*H₃*). ¹³C NMR (75.5 MHz, CDCl₃): δ = 170.0, 168.8 (CO ester), 154.7 (CO carbamate), 72.5 (CH₂O), 72.1 (C-4), 69.1 (C-3), 68.7 (C-2), 66.2 (C-6), 60.5 (C-1), 51.3 (C-5), 31.7-14.0 (*C*H₃CO, CH₂, CH₂*C*H₃). ESIMS: *m*/*z* = 545.18 [M + Na]⁺. Anal. calcd. for C₂₁H₃₄N₂O₁₁S: C 48.27, H 6.56, N 5.36, S 6.14. Found: C 48.09, H 6.43, N 5.18.

### EXAMPLE 16. Synthesis of compound 16.

Compound **16** was obtained by treatment of a solution of **15** (60 mg, 0.186 mmol) in MeOH (1.8 mL) with 1 M NaOMe in MeOH (73 µL), followed by neutralization with Amberlite^{®} IR120 hydrogen form, filtration, evaporation and freeze drying. Yield: 73 mg (quantitative). R_{f} 0.30 (70:10:1 DCM-MeOH-H₂O). [α]_{D} +20.40 (*c* 1.0 in MeOH). ¹H NMR (300 MHz, CD₃OD): δ = 5.29 (d, 1 H, *J*_{1,2} = 4.7 Hz, H-1), 4.50 (t, 1H, *J*_{6a,6b} = *J*_{5,6a} = 8.7 Hz, H-6a), 4.30 (dd, 1 H, *J*_{5,6b} = 4.8 Hz, H-6b), 4.17 (m, 2 H, CH₂O), 3.78 (m, 1 H, H-5), 3.57 (m, 2 H, H-2, H-3), 3.32 (m, 1 H, H-4),1.73-0.92 (m, 15 H, CH₂, CH₂C*H₃*). ¹³C NMR (75.5 MHz, CD₃OD): δ 156.4 (CO), 73.7 (C-4), 72.8 (C-2), 70.6 (CH₂O) 69.9 (C-3), 66.3 (C-6), 63.2 (C-1), 53.1 (C-5), 31.5-13.0 (CH₂, CH₂*C*H₃). ESIMS: m/z = 419.14 [M + Na]⁺. Anal. calcd. for C₁₅H₂₈N₂O₈S: C 45.44, H 7.12, N 7.07, S 8.09. Found: C 45.16, H 6.99, N 6.81.

### EXAMPLE 17. Synthesis of compound 17.

To a solution of **35** (100 mg, 0.267 mmol) in dry DCM (12 mL), O-dodecylsulfamate (0.320 mmol) and BF₃·OEt₂ (66 µL, 0.534 mmol) were added under Ar atmosphere at 0 °C. The mixture was stirred for 16 h. After complete formation of the product (TLC), Et₃N (0.3 mL) was added and the mixture was stirred for 10 min and concentrated. The resulting residue was purified by column chromatography, eluent (1:3 → 1:2 EtOAc-cyclohexane), to afford **17**. Yield: 75 mg (50%). *R_{f}* 0.43 (1:1 EtOAc-cyclohexane). [α]_{D} +28.58 (c 1.0 in DCM). ¹H NMR (300 MHz, CDCl₃): δ 5.69 (d, 1 H, *J*_{1,NH} = 3.7 Hz, NH), 5.63 (dd, 1 H, *J*_{1,2} = 5.0 Hz, H-1), 5.46 (t, 1 H, *J*_{2,3} = *J*_{3,4} = 9.8 Hz, H-3), 5.13 (dd, 1 H, H-2), 5.02 (t, 1 H, *J*_{4,5} = 9.5 Hz, H-4), 4.47 (t, 1 H, *J*_{6a,6b} = *J*_{5,6a} = 8.5 Hz, H-6a), 4.30 (bdd, 1 H, *J*_{5,6b} = 6.2 Hz, H-6b), 4.23 (m, 2 H, CH₂O) 4.13 (m, 1 H, H-5), 2.13, 2.09, 2.06 (3 s, 9 H, CH₃CO), 1.75-0.89 (m, 23 H, CH₂, CH₂C*H₃*). ¹³C NMR (75.5 MHz, CDCl₃): δ 170.0, 168.7 (CO ester), 154.7 (CO carbamate), 72.7 (CH₂O), 72.1 (C-4), 69.1 (C-3), 68.7 (C-2), 66.2 (C-6), 60.6 (C-1), 51.3 (C-5), 31.8-14.1 (*C*H₃CO, CH₂, CH₂*C*H₃). ESIMS: *m*/*z=* 601.2 [M + Na]⁺. Anal. calcd. for C₂₅H₄₂N₂O₁₁S: C 51.89, H 7.32, N 4.48, S 5.54. Found: C 52.10, H 7.46, N 4.74.

### EXAMPLE 18. Synthesis of compound 18.

Compound **18** was obtained by treatment of a solution of **17** (60 mg, 0.103 mmol) in MeOH (3 mL) with 1 M NaOMe in MeOH (31 µL), followed by neutralization with Amberlite^{®} IR120 hydrogen form, filtration, evaporation and freeze drying. Yield: 44 mg (quantitative). *R_{f}* 0.5 (80:10:1 DCM-MeOH-H₂O). [α]_{D} +30.7 (c 1.0 in MeOH). ¹H NMR (300 MHz, CD₃OD): δ 5.29 (d, 1 H, *J*_{1,2} = 4.7 Hz, H-1), 4.50 (t, 1H, *J*_{6a,6b} = *J*_{5,6a} = 8.7 Hz, H-6a), 4.30 (dd, 1 H, *J*_{5,6b} = 4.8 Hz, H-6b), 4.17 (m, 2 H, CH₂O), 3.78 (m, 1 H, H-5), 3.57 (m, 2 H, H-2, H-3), 3.32 (m, 1 H, H-4), 1.73-0.91 (m, 23 H, CH₂, CH₂C*H₃*). ¹³C NMR (75.5 MHz, CD₃OD): δ 156.3 (CO), 73.7 (C-4), 72.8 (C-2), 70.6 (CH₂O) 69.9 (C-3), 66.3 (C-6), 63.2 (C-1), 53.1 (C-5), 31.6-13.0 (CH₂, CH₂*C*H₃). ESIMS: m/z = 475.21 [M + Na]⁺. Anal. calcd. for C₁₉H₃₆N₂O₈S: C 50.43, H 8.02, N 6.19, S 7.08. Found: C 50.41, H 7.93, N 6.06.

### EXAMPLE 19. Synthesis of compound 19.

To a solution of **35** (100 mg, 0.267 mmol) in dry DCM (12 mL), O-hexadecylsulfamate (0.320 mmol) and BF₃·OEt₂ (66 µL, 0.534 mmol) were added under Ar atmosphere at 0 °C. The mixture was stirred for 16 h. After complete formation of the product (TLC), Et₃N (0.3 mL) was added, and the mixture was stirred for 10 min and concentrated. The resulting residue was purified by column chromatography, eluent (1:2 EtOAc-cyclohexane), to afford **19**. Yield: 111 mg (65%). *R_{f}* 0.70 (1:1 EtOAc-cyclohexane). [α]_{D} +27.13 (c 1.0 in DCM). ¹H NMR (300 MHz, CDCl₃): δ 5.84 (bs, 1 H, NH), 5.55 (d, 1 H, *J*_{1,2} = 5.0 Hz, H-1), 5.38 (t, 1 H, *J*_{2,3} = *J*_{3,4} = 9.9 Hz, H-3), 5.04 (dd, 1 H, H-2), 4.94 (t, 1 H, *J*_{4,5} = 9.5 Hz, H-4), 4.39 (t, 1 H, *J*_{6a,6b} = *J*_{5,6a} = 8.5 Hz, H-6a), 4.22 (bdd, 1 H, *J*_{5,6b} = 6.2 Hz, H-6b), 4.14 (m, 2 H, CH₂O) 4.05 (m, 1 H, H-5), 2.04, 2.00, 1.97 (3 s, 9 H, CH₃CO), 1.67-0.80 (m, 33 H, CH₂, CH₂C*H₃*). ¹³C NMR (75.5 MHz, CDCl₃): δ 170.0, 168.8 (CO ester), 154.7 (CO carbamate), 72.5 (CH₂O), 72.1 (C-4), 69.1 (C-3), 68.7 (C-2), 66.2 (C-6), 60.5 (C-1), 51.3 (C-5), 31.9-14.1 (*C*H₃CO, CH₂, CH₂*C*H₃). ESIMS: *m*/*z* = 657.3 [M + Na]⁺. Anal. calcd. for C₂₉H₅₀N₂O₁₁S: C 54.87, H 7.94, N 4.41, S 5.05. Found: C 55.11, H 8.17, N 4.26.

### EXAMPLE 20. Synthesis of compound 20.

Compound **20** was obtained by treatment of a solution of **19** (111 mg, 0.174 mmol) in MeOH (3 mL) with 1 M NaOMe in MeOH (31 µL), followed by neutralization with Amberlite^{®} IR120 hydrogen form, filtration, evaporation and freeze drying. Yield: 88 mg (quantitative). *R_{f}* 0.60 (80:10:1 DCM-MeOH-H₂O). [α]_{D} +24.7 (c 1.0 in MeOH). ¹H NMR (300 MHz, 1:1 CDCl₃-CD₃OD): δ = 5.22 (d, 1 H, *J*_{1,2} = 4.9 Hz, H-1), 4.40 (t, 1H, *J*_{6a,6b} = *J*_{5,6a} = 8.8 Hz, H-6a), 4.20 (dd, 1 H, *J*_{5,6b} = 4.8 Hz, H-6b), 4.09 (m, 2 H, CH₂O), 3.70 (m, 1 H, H-5), 3.47 (m, 2 H, H-2, H-3), 3.22 (m, 1 H, H-4), 1.64-0.79 (m, 33 H, CH₂, CH₂C*H₃*). ¹³C NMR (75.5 MHz, 1:1 CDCl₃-CD₃OD): δ 156.3 (CO), 73.7 (C-4), 73.0 (C-2), 71.1 (CH₂O), 69.8 (C-3), 66.4 (C-6), 63.1 (C-1), 53.0 (C-5), 31.7-13.6 (CH₂, CH₂*C*H₃). ESIMS: *m*/*z* = 531.27 [M + Na]⁺. Anal. calcd. for C₂₈H₄₄N₂O₈S: C 54.31, H 8.72, N 5.51, S 6.30. Found: C 54.08, H 8.43, N 5.19.

### EXAMPLE 21. Synthesis of compound 21.

To a solution of **35** (100 mg, 0.267 mmol) in dry DCM (12 mL), octylsulfonamide (0.320 mmol) and BF₃·OEt₂ (66 µL, 0.534 mmol) were added under Ar atmosphere at 0 ºC. The mixture was stirred for 16 h. After complete formation of the product (TLC), Et₃N (0.3 mL) was added, and the mixture was stirred for 10 min and concentrated. The resulting residue was purified by column chromatography (1:2 EtOAc-cyclohexane) to afford the sulphonamide derivative **21.** Yield: 123 mg (91%). R_{f} 0.40 (1:1 EtOAc-cyclohexane). [α]_{D} +41.9 (c 1.0 in DCM). ¹H NMR (300 MHz, CDCl₃): δ 5.96 (bs, 1 H, *J*_{NH,1} = 6.1 Hz, NH), 5.52 (bt, 1 H, *J*_{1,2} = 5.4 Hz, H-1), 5.45 (t, 1 H, *J*_{2,3} = *J*_{3,4} = 9.9 Hz, H-3), 5.01 (dd, 1 H, H-2), 4.92 (t, 1 H, *J*_{4,5} = 9.5 Hz, H-4), 4.40 (t, 1 H, *J*_{6a,6b} = *J*_{5,6a} = 8.5 Hz, H-6a), 4.22 (bdd, 1 H, *J*_{5,6b} = 6.2 Hz, H-6b), 4.06 (m, 1 H, H-5), 3.14 (m, 2 H, CH₂S), 2.04, 2.00, 1.99 (3 s, 9 H, CH₃CO), 1.68-0.80 (m, 17 H, CH₂, CH₂C*H₃*).¹³C NMR (75.5 MHz, CDCl₃): δ 170.0, 169.3 (CO ester), 154.4 (CO carbamate), 72.3 (C-4), 68.8 (C-3), 68.7 (C-2), 66.9 (C-6), 59.5 (C-1), 52.7 (CH₂S), 52.0 (C-5), 31.6-14.0 (*C*H₃CO, CH₂, CH₂*C*H₃). ESIMS: *m*/*z=* 529.1 [M + Na]⁺. Anal. calcd. for C₂₁H₃₄N₂O₁₀S: C 49.79, H 6.77, N 5.53, S 6.33. Found: C 50.03, H 6.87, N 5.37, S 6.21.

### EXAMPLE 22. Synthesis of compound 22.

Compound **22** was obtained by treatment of a solution of **21** (123 mg, 0.243 mmol) in MeOH (2.4 mL) with 1 M NaOMe in MeOH (100 µL), followed by neutralization with Amberlite^{®} IR120 hydrogen form, filtration, evaporation and freeze drying. Yield: 92 mg (quantitative). *R_{f}* 0.4 (70:10:1 DCM-MeOH-H₂O). [α]_{D} +33.04 (c 1.0 in MeOH).¹H NMR (300 MHz, CD₃OD) δ = 5.21 (d, 1 H, *J*_{1,2} = 4.9 Hz, H-1), 4.42 (t, 1 H, *J*_{2,3} = *J*_{3,4} = 8.8 Hz, H-3), 4.19 (dd, 1 H, H-2), 3.73 (m, 1 H, H-5), 3.48 (m, 2 H, *J*_{6a,6b} = *J*_{5,6a} = 8.7 Hz, H-6a, H-4), 3.25 (m, 1 H, H-6b), 3.07 (m, 2 H, CH₂S), 1.71-0.79 (m, 17 H, CH₂, CH₂C*H₃*).¹³C NMR (75.5 MHz, CD₃OD): δ 156.6 (CO), 73.7 (C-4), 73.0 (C-2), 69.9 (C-3), 66.7 (C-6), 62.5 (C-1), 53.1 (C-5), 52.1 (CH₂S), 31.5-13.5 (CH₂, CH₂*C*H₃). ESIMS: *m*/*z* = 403.15 [M + Na]⁺. Anal. calcd. for C₁₅H₂₈N₂O₇S: C 47.36, H 7.42, N 7.36, S 8.43. Found C 47.17, H 7.28, N 7.15, S 8.20.

### EXAMPLE 23. Synthesis of compound 23.

Compound **23** was obtained by conjugation of isothiocyanate derivative **39** and octylamine. Precursor **39** was prepared from **35** according to Scheme 3, through the procedure described hereinafter.

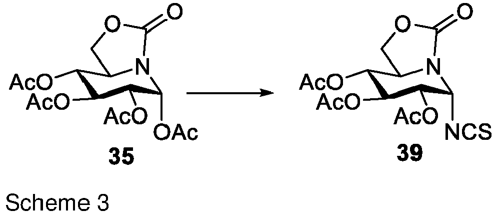

To a solution of **35** (100 mg, 0.27 mmol) and SnCl₄ (1 m in DCM, 270 µL) in DCM (4 mL) was stirred for 5 min, then trimethylsilyl isothiocyanate (42 µL, 0.29 mmol) was added and the reaction mixture was stirred at room temperature for 60 min. Saturated aqueous solution of NaHCO₃ (2 × 45 mL) was added and the aqueous phase was extracted with DCM (3 × 45 mL). The organic layer was washed with water (30 mL), dried (MgSO₄), filtered and concentrated. The resulting residue was purified by column chromatography (1:1 EtOAc-cyclohexane) to afford **39.** Yield: 80 mg (80%). R_{f} 0.67 (1:1 EtOAc-petroleum ether). [α]_{D} +79.6 (c 1.0 in CHCl₃). ¹H NMR (500 MHz, CDCl₃) δ 6.09 (d, 1 H, J_{1,2} = 4.5 Hz, H-1), 5.51 (t, 1 H, J_{2,3} = J_{3,4} = 10.0 Hz, H-3), 5.02 (dd, 1 H, H-2), 4.95 (t, 1 H, J_{4,5} = 10.0 Hz, H-4), 4.49 (dd, 1 H, J_{6a,6b} = 9.5 Hz, J_{5,6a} = 8.0 Hz, H-6a), 4.30 (dd, 1 H, J_{5,6b} = 8.0 Hz, H-6b), 4.03 (dt, 1 H, H-5), 2.17-2.09 (3 s, 9 H, MeCO). ¹³C NMR (125.7 MHz, CDCl₃) δ 170.0-169.5 (MeCO), 154.4 (CO), 145.2 (CS), 71.8 (C-4), 70.2 (C-2), 69.0 (C-3), 66.8 (C-6), 63.3 (C-1), 52.3 (C-5), 20.5-20.3 (MeCO). ESIMS: *m*/*z* 394.9 [M + Na]⁺. Anal. Calcd for C₁₄H₁₆N₂O₈S: C 45.16, H 4.33, N 7.52, S 8.61. Found: C 45.15, H 4.22, N 7.26, S 8.33.

In a subsequent step, to a stirred solution of the isothiocyanate derivative **39** (300 mg, 0.81 mmol) in anhydrous DCM (20 mL), n-octylamine (133 µL, 0.81 mmol) was added and the reaction mixture was stirred for 90 min at room temperature. The solvent was removed under reduced pressure and the resulting residue was purified by column chromatography (1:2 → 2:1 EtOAc-cyclohexane) to afford **23.** Yield: 370 mg (92%). R*_{f}* 0.27 (1:1 EtOAc-cyclohexane). [α]_{D} +64.0 (c 1.0, CHCl₃). ¹H NMR (500 MHz, CDCl₃) δ 7.46 (bs, 1 H, NH), 6.43 (d, 1 H, *J*_{NH,1} = 5.0 Hz, NH), 5.69 (t, 1 H, *J*_{1,2} = 5.0 Hz, H-1), 5.45 (dd, 1 H, *J*_{2,3} = 10.0 Hz, *J*_{3,4} = 9.0 Hz, H-3), 5.07 (dd, 1 H, H-2), 4.96 (t, 1 H, *J*_{4,5} = 9.0 Hz, H-4), 4.54 (t, 1 H, *J*_{6a,6b} = *J*_{5,6a} = 9.0 Hz, H-6a), 4.39 (dd, 1 H, *J*_{5,6b} = 7.5 Hz, H-6b), 4.06 (bq, 1 H, H-5), 3.61-3.55 (m, 2 H, NHC*H*₂), 2.16-2.09 (3 s, 9 H, MeCO), 1.75-1.60 (m, 2 H, NHCH₂C*H*₂), 1.40-1.22 (m, 10 H, CH₂), 0.90 (t, 3 H, ³*J*_{H,H} = 7.0 Hz, CH₃). ¹³C NMR (125.7 MHz, CDCl₃) δ 181.9 (CS), 170.0-169.4 (MeCO), 157.1 (CO), 72.4 (C-4), 68.4 (C-3), 68.2 (C-2), 67.5 (C-6), 58.4 (C-1), 51.8 (C-5), 46.9 (NHCH₂), 31.8-22.6 (CH₂), 20.6-20.4 (MeCO), 14.1 (CH₃). ESIMS: *m*/*z* 524.0 ([M + Na]+). Anal. Calcd for C₂₂H₃₅N₈O₈S: C 52.68, H 7.03, N 8.38, S 6.39. Found: C 52.49, H 6.80, N 8.12, S 6.09.

### EXAMPLE 24. Synthesis of compound 24.

Compound **24** was obtained from the carbodiimide derivative **40** and LiAIHSeH. Precursor **40** was prepared from compound **23** according to Scheme 4, through the procedure described hereinafter.

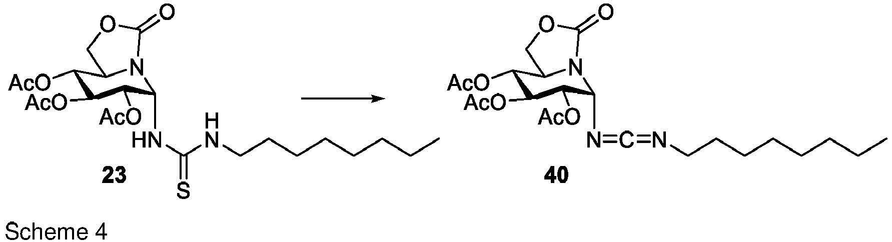

To a stirred solution of the α-glycosyl thiourea 23 (170 mg, 0.34 mmol) in DCM-H₂O (1:1, 8 mL), HgO (220 mg, 1.02 mmol) was added, and the reaction mixture was stirred at room temperature for 2 h. Then, DCM (20 mL) was added and the organic layer was separated, dried (MgSO₄), filtered through a pad of Celite and concentrated. The resulting residue was purified by column chromatography (1:1 EtOAc-cyclohexane) to afford **40.** Yield: 110 mg (70%). R*_{f}* 0.73 (1:1 EtOAc-cyclohexane).

[α]_{D} +41.7 (c 1.0, CHCl₃). ¹H NMR (500 MHz, CDCl₃) δ 5.72 (d, 1 H, *J*_{1,2} = 4.5 Hz, H-1), 5.53 (t, 1 H, *J*_{2,3} = *J*_{3,4} = 9.5 Hz, H-3), 4.97 (dd, 1 H, H-2), 4.92 (t, 1 H, *J*_{4,5} = 9.5 Hz, H-4), 4.43 (t, 1 H, *J*_{6a,6b} = *J*_{5,6a} = 8.5 Hz, H-6a), 4.25 (t, 1 H, *J*_{5,6b} = 8.5 Hz, H-6b), 4.07 (bq, 1 H, H-5), 3.36-3.25 (m, 2 H, NCH₂), 2.12-2.06 (3 s, 9 H, MeCO), 1.65-1.58 (m, 2 H, NCH₂C*H*₂), 1.41-1.24 (m, 10 H, CH₂), 0.90 (t, 3 H, ³*J*_{H,H} = 7.0 Hz, CH₃). ¹³C NMR (125.7 MHz, CDCl₃) δ 170.1-169.5 (MeCO), 155.1 (CO), 137.0 (NCN), 72.4 (C-4), 70.9 (C-2), 69.3 (C-3), 66.7 (C-6), 63.0 (C-1), 52.0 (C-5), 46.4 (NCH₂), 31.8-22.6 (CH₂), 20.6-20.5 (MeCO), 14.1 (CH₃). ESIMS: *m*/*z* 490.0 ([M + Na]⁺). Anal. Calcd for C₂₂H₃₃N₃O₈: C 56.52, H 7.11, N 8.99. Found: C 56.58, H 7.03, N 8.76.

In a subsequent step, to a solution of the octylcarbodiimide **40** (90 mg, 0.19 mmol) in anhydrous THF (1 mL), under Ar atmosphere, 1 N dry hydrogen chloride in diethyl ether (0.2 mL) was added, and the misxture was stirred at room temperature for 4 h. Then, an anydrous THF solution (9 mL) of LiAIHSeH (0.96 mmol), prepared in situ, was added at 0 °C to the previous mixture, and the reaction mixture was stirred for 15 min, diluted with diethyl ether (50 mL), washed with brine (10 mL), dried (MgSO₄), filtered and concentrated. The resulting residue was purified by column chromatography (1:3 EtOAc-cyclohexane) to afford **24.** Yield: 79 mg (76%). R*_{f}* 0.38 (1:1 EtOAc-cyclohexane). [α]_{D} +72.2 (c 1.2 in DCM). ¹H NMR (300 MHz, CDCl₃) δ 7.73 (bs, 1 H, NH), 6.80 (bs, 1 H, NH), 5.58 (t, 1 H, *J*_{1,2} = *J*_{1,NH} = 6.0 Hz, H-1), 5.41 (t, 1 H, *J*_{2,3} = *J*_{3,4} = 9.5 Hz, H-3), 5.02 (dd, 1 H, H-2), 4.91 (t, 1 H, *J*_{4,5} = 9.0 Hz, H-4), 4.47 (t, 1 H, *J*_{6a,6b} = *J*_{5,6a} = 9.0 Hz, H-6a), 4.32 (dd, 1 H, *J*_{5,6b} = 7.5 Hz, H-6b), 4.03 (bq, 1 H, H-5), 3.56 (dt, 2 H, ²*J*_{H,H} = 12.3 Hz, ³*J*_{H,H} = 7.5 Hz, NHC*H*₂), 2.09-2.000 (3 s, 9 H, MeCO), 1.70-1.50 (m, 2 H, NHCH₂C*H*₂), 1.35-1.10 (m, 10 H, CH₂), 0.81 (t, 3 H, ³*J*_{H,H} = 7.0 Hz, CH₃). ¹³C NMR (75.5 MHz, CDCl₃) δ 180.7 (CSe), 170.0-169.3 (MeCO), 157.2 (CO), 72.3 (C-4), 68.3 (C-3), 68.0 (C-2), 67.7 (C-6), 58.6 (C-1), 51.9 (C-5), 50.0 (NHCH₂), 31.7-22.6 (CH₂), 20.6-20.4 (MeCO), 14.1 (CH₃). ESIMS: *m*/*z* 550.28 [M + H]⁺. Anal. Calcd for C₂₂H₃₅N₃O₈Se: C 48.17, H 6.43, N 7.66. Found: C 48.04, H 6.29, N 7.45.

### EXAMPLE 25. Synthesis of compound 25.

Compound **25** was obtained by treatment of a solution of **24** (50 mg, 0.09 mmol) in MeOH (2 mL) with 1 M NaOMe in MeOH (50 µL), followed by neutralization with Amberlite^{®} IR120 hydrogen form, filtration, evaporation and purification by column chromatography (8:1 DCM-MeOH). Yield: 31 mg (82%). R*_{f}* 0.56 (8:1 DCM-MeOH). ¹H NMR (300 MHz, (CD₃)₂SO) δ 8.02 (bt, 1 H, *J =* 5.4 Hz, NH), 7.91 (d, 1 H, *J*_{NH,1} = 7.0 Hz, NH), 5.89 (bs, 1 H, H-1), 5.48 (d, 1 H, *J*_{OH,4} = 4.7 Hz, OH), 5.40 (d, 1 H, *J*_{OH,2} = 3.1 Hz, OH), 5.07 (d, 1 H, *J*_{OH,3} = 3.4 Hz, OH), 4.26 (t, 1 H, *J*_{6a,6b} = *J*_{5,6a} = 8.3 Hz, H-6a), 4.08 (dd, 1 H, J_{5,6b} = 3.0 Hz, H-6b), 3.50-3.29 (m, 5 H, H-2, H-3, H-5, NHCH₂), 3.05 (td, 1 H, *J*_{3,4} = *J*_{4,5} = 9.0 Hz, H-4), 1.50-1.35 (m, 2 H, CH₂), 1.30-1.10 (m, 10 H, CH₂), 0.79 (t, 3 H, ³*J*_{H,H} = 6.8 Hz, CH₃). ¹³C NMR (75.5 MHz, (CD₃)₂SO) δ 183.8 (C=Se), 155.6 (CO), 73.4-73.3 (C-4, C-3), 70.3 (C-2), 65.9 (C-6), 64.9 (C-1), 54.2 (C-5), 47.0 (NHCH₂), 31.7-22.5 (CH₂), 14.4 (CH₃). ESIMS: *m*/*z* 424.27 [M + H]⁺.

### EXAMPLE 26. Synthesis of compound 26.

To a stirred solution of the isothiocyanate derivative **39** (372 mg, 1.00 mmol) in DCM (28 mL), n-dodecylamine (185 mg, 1.00 mmol) was added and the reaction mixture was stirred for 15 min at room temperature. The solvent was removed under reduced pressure and the resulting residue was purified by column chromatography (1:2 EtOAc-cyclohexane) to afford **26.** Yield: 490 mg (88%). R*_{f}* 0.46 (1:1 EtOAc-cyclohexane). [α]_{D} +65.3 (c 1.1 in DCM). ¹H NMR (300 MHz, CDCl₃) δ 7.43 (bs, 1 H, NH), 6.40 (d, 1 H, *J*_{NH,1} = 6.0 Hz, NH), 5.67 (t, 1 H, *J*_{1,2} = 6.0 Hz, H-1), 5.43 (t, 1 H, *J*_{2,3} = *J*_{3,4} = 9.5 Hz, H-3), 5.05 (dd, 1 H, H-2), 4.94 (t, 1 H, *J*_{4,5} = 9.5 Hz, H-4), 4.52 (t, 1 H, *J*_{6a,6b} = *J*_{5,6a} = 9.0 Hz, H-6a), 4.36 (dd, 1 H, *J*_{5,6b} = 7.6 Hz, H-6b), 4.03 (bq, 1 H, H-5), 3.59-3.50 (m, 2 H, NHCH₂), 2.13-2.06 (3 s, 9 H, MeCO), 1.68-1.54 (m, 2 H, NHCH₂CH₂), 1.36-1.18 (m, 18 H, CH₂), 0.87 (t, 3 H, ³*J*_{H,H} = 7.0 Hz, CH₃). ¹³C NMR (75.5 MHz, CDCl₃) δ 181.9 (CS), 170.0-169.3 (MeCO), 157.1 (CO), 72.4 (C-4), 68.3 (C-3), 68.2 (C-2), 67.6 (C-6), 58.4 (C-1), 51.8 (C-5), 47.0 (NHCH₂), 31.9-22.7 (CH₂), 20.6-20.4 (MeCO), 14.1 (CH₃). ESIMS: *m*/*z* 580.3 [M + Na]⁺. Anal. Calcd for C₂₆H₄₃N₃O₈S: C 55.99, H 7.77, N 7.53, S 5.75. Found: C 55.67, H 7.55, N 7.21, S 5.41.

### EXAMPLE 27. Synthesis of compound 27.

Compound **27** was obtained by treatment of a solution of **26** (27 mg, 0.05 mmol) in MeOH (0.7 mL) with 1 M NaOMe in MeOH (20 µL), followed by neutralization with Amberlite^{®} IR120 hydrogen form, filtration, evaporation and freeze drying. Column chromatography (6:1 → 4:1 EtOAc-MeOH). Yield: 19.4 mg (94%). R*_{f}* 0.56 (4:1 EtOAc-MeOH). [α]_{D} -21.3 (c 0.9 in DMSO). ¹H NMR (300 MHz, DMSO-d₆) δ 8.14-7.99 (m, 2 H, NH), 5.99 (t, 1 H, *J*_{1,2} = *J*_{1,NH} = 6.0 Hz, H-1), 5.60 (d, 1 H, *J*_{OH,4} = 4.5 Hz, OH), 5.34 (d, 1 H, J_{OH,2} = 4.5 Hz, OH), 5.17 (d, 1 H, *J*_{OH,3} = 4.2 Hz, OH), 4.29 (t, 1 H, *J*_{6a,6b} = *J*_{5,6a} = 8.5 Hz, H-6a), 4.16 (dd, 1 H, *J*_{5,6b} = 2.7 Hz, H-6b), 3.56-3.28 (m, 5 H, H-2, H-3, H-5, NHCH₂), 3.09 (td, 1 H, *J*_{3,4} = *J*_{4,5} = 9.0 Hz, H-4), 1.60-1.20 (m, 20 H, CH₂), 0.86 (t, 3 H, ³*J*_{H,H} = 6.5 Hz, CH₃). ¹³C NMR (75.5 MHz, DMSO-d₆) v 184.1 (C=S), 155.5 (CO), 73.5 (C-4), 73.2 (C-3), 70.5 (C-2), 65.6 (C-6), 63.3 (C-1), 54.1 (C-5), 44.3 (NHCH₂), 31.8-22.5 (CH₂), 14.4 (CH₃). ESIMS: *m*/*z* 454.4 [M + Na]⁺. Anal. Calcd for C₂₀H₃₇N₃O₅S: C 55.66, H 8.64, N 9.74, S 7.43. Found: C 55.31, H 8.29, N 9.40, S 7.06.

### EXAMPLE 28. Synthesis of compound 28.

Compound **28** was obtained from the carbodiimide derivative **41** and LiAIHSeH. Precursor **41** was prepared from compound **26** according to Scheme 5, through the procedure described hereinafter.

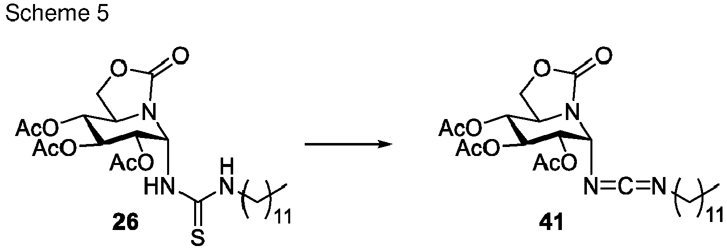

To a stirred solution of the α-glycosyl thiourea **26** (490 mg, 0.88 mmol) in DCM-H₂O (1:1, 21 mL), HgO (572 mg, 2.64 mmol) was added, and the reaction mixture was stirred at room temperature for 1 h. Then, DCM (20 mL) was added and the organic layer was separated, dried (MgSO₄), filtered through a pad of Celite and concentrated. The resulting residue was purified by column chromatography (1:1 EtOAc-cyclohexane) to afford **41.** Yield: 423 mg (92%). R*_{f}* 0.33 (1:1 EtOAc-cyclohexane). [α]_{D} +52.0 (c 1.0 in DCM). ¹H NMR (300 MHz, CDCl₃) δ 5.63 (d, 1 H, *J*_{1,2} = 4.2 Hz, H-1), 5.43 (t, 1 H, *J*_{2,3} = *J*_{3,4} = 10.0 Hz, H-3), 4.92 (dd, 1 H, H-2), 4.88 (t, 1 H, *J*_{4,5} = 10.0 Hz, H-4), 4.34 (dd, 1 H, *J*_{6a,6b} = 9.0 Hz, *J*_{5,6a} = 8.1 Hz, H-6a), 4.18 (t, 1 H, *J*_{5,6b} = 9.0 Hz, H-6b), 4.09-3.92 (m, 1 H, H-5), 3.30-3.13 (m, 2 H, NCH₂) 2.03-1.97 (3 s, 9 H, MeCO), 1.59-1.46 (m, 2 H, NCH₂CH₂), 1.30-1.15 (m, 18 H, CH₂), 0.81 (t, 3 H, ³*J*_{H,H} = 7.0 Hz, CH₃). ¹³C NMR (75.5 MHz, CDCl₃) δ 169.9-169.3 (MeCO), 154.9 (CO), 136.8 (NCN), 72.1 (C-4), 70.6 (C-2), 69.1 (C-3), 66.5 (C-6), 62.8 (C-1), 51.8 (C-5), 46.2 (NCH₂), 31.7-22.5 (CH₂), 20.3 (MeCO), 13.9 (CH₃). ESIMS: *m*/*z* 546.4 [M + Na]⁺. HRFABMS Calcd for C₂₆H₄₁N₃O₈SNa [M + Na]⁺ 546.2786, found 546.2765.

In a subsequent step, to a solution of the α-dodecyicarbodiimide **41** (96 mg, 0.18 mmol) in anhydrous THF (1 mL), under Ar atmosphere, 1 N HCI in Et₂O (0.18 mL) was added and the mixture was stirred at room temperature for 4 h. Then, a solution of LiAIHSeH (0.92 mmol) in THF (9 mL), prepared in situ, was added at 0 °C to the previous mixture, and the resulting reaction was stirred for 90 min, diluted with diethyl ether (50 mL), washed with brine (10 mL), dried (MgSO₄), filtered and concentrated. The resulting residue was purified by column chromatography (1:3 EtOAc-cyclohexane) to afford **28.** Yield: 51 mg (47%). R*_{f}* 0.47 (1:1 EtOAc-cyclohexane). [α]_{D} +55.0 (c 1.3 in DCM). ¹H NMR (300 MHz, CDCl₃) δ 7.81 (bs, 1 H, NH), 6.74 (d, 1 H, *J*_{NH,1} = 6.0 Hz, NH), 5.61 (t, 1 H, *J*_{1,2} = 6.0 Hz, H-1), 5.43 (dd, 1 H, *J*_{2,3} = 10.0 Hz, *J*_{3,4} = 9.0 Hz, H-3), 5.04 (dd, 1 H, H-2), 4.92 (t, 1 H, *J*_{4,5} = 9.0 Hz, H-4), 4.53 (t, 1 H, *J*_{6a,6b} = *J*_{5,6a} = 9.3 Hz, H-6a), 4.38 (dd, 1 H, *J*_{5,6b} = 7.5 Hz, H-6b), 4.03 (bq, 1 H, H-5), 3.63 (dt, 2 H, ²*J*_{H,H} = 12.3 Hz, ³*J*_{H,H} = 7.5 Hz, NHCH₂), 2.16-2.07 (3 s, 9 H, MeCO), 1.70-1.55 (m, 2 H, NHCH₂CH₂), 1.35-1.20 (m, 18 H, CH₂), 0.88 (t, 3 H, ³*J*_{H,H} = 7.0 Hz, CH₃). ¹³C NMR (75.5 MHz, CDCl₃) δ 180.7 (CSe), 169.9-169.2 (MeCO), 157.2 (CO), 72.4 (C-4), 68.2 (C-3), 68.0 (C-2), 67.7 (C-6), 58.5 (C-1), 51.9 (C-5), 50.1 (NHCH₂), 31.9-22.7 (CH₂), 20.6-20.5 (MeCO), 14.1 (CH₃). ⁷⁷Se NMR (95.4 MHz, CDCl₃, 323 K) δ 240.4. ESIMS: *m*/*z* 628.2 [M + Na]⁺. Anal. Calcd for C₂₆H₄₃N₃O₈Se: C 51.65, H 7.17, N 6.95. Found: C 51.40, H 6.89, N 6.63.

### EXAMPLE 29. Synthesis of compound 29.

Compound **29** was obtained by treatment of a solution of **28** (20 mg, 0.03 mmol) in MeOH (0.7 mL) with 1 M NaOMe in MeOH (20 µL), followed by neutralization with Amberlite^{®} IR120 hydrogen form, filtration, evaporation and freeze drying. Column chromatography (6:1 DCM-MeOH). Yield: 13 mg (82%). R*_{f}* 0.67 (9:1 DCM-MeOH). [α]_{D} -11.2 (c 1.0 in DMSO). ¹H NMR (300 MHz, DMSO-d₆) δ 8.03 (t, 1 H, *J* = 4.7 Hz, NH), 7.94 (d, 1 H, *J*_{NH,1} = 7.2 Hz, NH), 5.95 (bs, 1 H, H-1), 5.57 (d, 1 H, *J*_{OH,4} = 5.0 Hz, OH), 5.50 (d, 1 H, *J*_{OH,2} = 3.3 Hz, OH), 5.16 (d, 1 H, *J*_{OH,3} = 3.9 Hz, OH), 4.32 (t, 1 H, *J*_{6a,6b} = *J*_{5,6a} = 8.1 Hz, H-6a), 4.14 (dd, 1 H, *J*_{5,6b} = 3.0 Hz, H-6b), 3.55-3.31 (m, 5 H, H-2, H-3, H-5, NHCH₂), 3.12 (td, 1 H, *J*_{3,4} = *J*_{4,5} = 9.0 Hz, H-4), 1.55-1.41 (m, 2 H, CH₂), 1.35-1.15 (m, 18 H, CH₂), 0.85 (t, 3 H, ³*J*_{H,H} = 7.0 Hz, CH₃). ¹³C NMR (75.5 MHz, DMSO-d₆) δ 183.3 (C=Se), 155.1 (CO), 72.9-72.8 (C-4, C-3), 69.8 (C-2), 65.4 (C-6), 64.4 (C-1), 53.7 (C-5), 46.5 (NHCH₂), 31.3-22.1 (CH₂), 13.9 (CH₃). ESIMS: *m*/*z* 478.1 [M - H]⁻. Anal. Calcd for C₂₀H₃₇N₃O₅Se: C 50.20, H 7.79, N 8.78. Found: C 49.91, H 7.55, N 8.66.

### EXAMPLE 30. Synthesis of compound 30.

To a stirred solution of the isothiocyanate derivative **39** (210 mg, 0.56 mmol) in DCM (14 mL), hexadecylamine (136 mg, 0.56 mmol) was added and the reaction mixture was stirred for 60 min at room temperature. The solvent was removed under reduced pressure and the resulting residue was purified by column chromatography (1:2 → 2:1 EtOAc-cyclohexane) to afford **30.** Yield: 265 mg (77%). R*_{f}* 0.47 (1:1 EtOAc-cyclohexane). [α]_{D} +41.4 (c 1.2, CH₂Cl₂). ¹H NMR (300 MHz, CDCl₃) δ 7.36 (bs, 1 H, NH), 6.35 (d, 1 H, *J*_{NH,1} = 6.2 Hz, NH), 5.61 (t, 1 H, *J*_{1,2} = 5.9 Hz, H-1), 5.38 (dd, 1 H, *J*_{2,3} = 10.0 Hz, *J*_{3,4} = 9.2 Hz, H-3), 5.00 (dd, 1 H, H-2), 4.89 (t, 1 H, *J*_{4,5} = 9.2 Hz, H-4), 4.56 (t, 1 H, *J*_{6a,6b} = *J*_{5,6a} = 9.0 Hz, H-6a), 4.30 (dd, 1 H, *J*_{5,6b} = 7.6 Hz, H-6b), 3.98 (bq, 1 H, H-5), 3.55-3.43 (m, 2 H, NHC*H*₂), 2.07-2.00 (3 s, 9 H, MeCO), 1.62-1.48 (m, 2 H, NHCH₂C*H*₂), 1.32-1.09 (m, 26 H, CH₂), 0.81 (t, 3 H, ³*J*_{H,H} = 6.5 Hz, CH₃). ¹³C NMR (75.5 MHz, CDCl₃) δ 181.9 (CS), 170.0-169.3 (MeCO), 157.1 (CO), 72.4 (C-4), 68.3 (C-3), 68.2 (C-2), 67.6 (C-6), 58.4 (C-1), 51.8 (C-5), 46.9 (NHCH₂), 31.9-22.7 (CH₂), 20.5-20.4 (MeCO), 14.1 (CH₃). ESIMS: *m*/*z* 614.54 ([M + H]⁺). Anal. Calcd for C₃₀H₅₁N₃O₈S: C 58.70, H 8.38, N 6.85, S 5.22. Found: C 58.81, H 8.47, N 6.75, S 5.03.

### EXAMPLE 31. Synthesis of compound 31.

Compound **31** was obtained from the carbodiimide derivative **42** and LiAIHSeH. Precursor **42** was prepared from compound **30** according to Scheme 6, through the procedure described hereinafter.

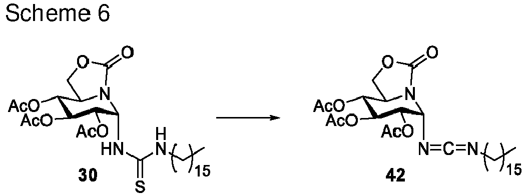

To a stirred solution of **30** (330 mg, 0.53 mmol) in DCM-H₂O (1:1, 14 mL), HgO (350 mg, 1.61 mmol) was added, and the reaction mixture was stirred at room temperature for 2 h. Then, DCM (20 mL) was added, and the organic layer was separated, dried (MgSO₄), filtered through a pad of Celite and concentrated. The resulting residue was purified by column chromatography (1:3 EtOAc-cyclohexane) to afford **42.** Yield: 260 mg (83%). R*_{f}* 0.73 (1:1 EtOAc-cyclohexane). [α]_{D} +37.0 (c 1.2, DCM). ¹H NMR (300 MHz, CDCl₃) δ 5.62 (d, 1 H, *J*_{1,2} = 4.4 Hz, H-1), 5.44 (t, 1 H, *J*_{2,3} = *J*_{3,4} = 9.8 Hz, H-3), 4.88 (dd, 1 H, H-2), 4.84 (t, 1 H, *J*_{4,5} = 9.5 Hz, H-4), 4.33 (t, 1 H, *J*_{6a,6b} = *J*_{5,6a} = 8.7 Hz, H-6a), 4.16 (t, 1 H, *J*_{5,6b} = 8.5 Hz, H-6b), 4.00 (bq, 1 H, H-5), 3.30-3.14 (m, 2 H, NCH₂), 2.03-1.97 (3 s, 9 H, MeCO), 1.57-1.45 (m, 2 H, NCH₂C*H*₂), 1.34-1.10 (m, 26 H, CH₂), 0.81 (t, 3 H, ³*J*_{H,H} = 7.0 Hz, CH₃). ¹³C NMR (75.5 MHz, CDCl₃) δ 170.1-169.5 (MeCO), 155.1 (CO), 137.0 (NCN), 72.4 (C-4), 70.9 (C-2), 69.3 (C-3), 66.7 (C-6), 63.0 (C-1), 52.0 (C-5), 46.4 (NCH₂), 31.9-22.7 (CH₂), 20.5 (MeCO), 14.1 (CH₃). ESIMS: *m*/*z* 602.48 ([M + Na]⁺).

In a subsequente step, to a solution of the α-hexadecylcarbodiimide **42** (180 mg, 0.31 mmol) in anhydrous THF (1.6 mL) under Ar atmosphere, 1 N dry hydrogen chloride in diethyl ether (319 µL) was added and the mixture was stirred at room temperature for 4 h. Then, an anydrous THF solution (15 mL) of LiAIHSeH (1.55 mmol), prepared in situ, was added at 0 °C to the previous mixture, and the resulting reaction mixture was stirred for 15 min, diluted with diethyl ether (50 mL), washed with brine (10 mL), dried (MgSO₄), filtered and concentrated. The resulting residue was purified by column chromatography (1:2 → 1:1 EtOAc-cyclohexane) to afford **31.** Yield: 100 mg (49%). R*_{f}* 0.47 (1:1 EtOAc-cyclohexane). [α]_{D} +54.4 (c 1.2 in DCM). ¹H NMR (300 MHz, CDCl₃) δ 7.73 (bs, 1 H, NH), 6.74 (d, *J*_{1,NH} = 6.1 Hz 1 H, NH), 5.56 (t, 1 H, *J*_{1,2} = 6.1 Hz, H-1), 5.38 (t, 1 H, *J*_{2,3} = *J*_{3,4} = 10.0 Hz, H-3), 5.00 (dd, 1 H, H-2), 4.88 (t, 1 H, *J*_{4,5} = 9.0 Hz, H-4), 4.46 (t, 1 H, *J*_{6a,6b} = *J*_{5,6a} = 9.0 Hz, H-6a), 4.31 (dd, 1 H, *J*_{5,6b} = 7.6 Hz, H-6b), 3.97 (bq, 1 H, H-5), 3.56 (dt, 2 H, ²*J*_{H,H} = 12.3 Hz, ³*J*_{H,H} = 7.4 Hz, NHC*H*₂), 2.09-2.00 (3 s, 9 H, MeCO), 1.64-1.51 (m, 2 H, NHCH₂C*H*₂), 1.33-1.11 (m, 26 H, CH₂), 0.81 (t, 3 H, ³*J*_{H,H} = 7.0 Hz, CH₃). ¹³C NMR (75.5 MHz, CDCl₃) δ 180.8 (C=Se), 170.0-169.2 (MeCO), 157.2 (CO), 72.4 (C-4), 68.3 (C-3), 68.0 (C-2), 67.7 (C-6), 58.6 (C-1), 51.9 (C-5), 50.1 (NHCH₂), 31.9-22.7 (CH₂), 20.6-20.4 (MeCO), 14.1 (CH₃). ESIMS: *m*/*z* 662.41 [M + H]⁺.

### EXAMPLE 32. Synthesis of compound 32.

Compound **32** was obtained by treatment of a solution of **31** (73 mg, 0.11 mmol) in MeOH (0.7 mL) with 1 M NaOMe in MeOH (20 µL), followed by neutralization with Amberlite^{®} IR120 hydrogen form, filtration, evaporation and freeze drying. Column chromatography (8:1 → 7:1 DCM-MeOH). Yield: 48 mg (81%). R*_{f}* 0.47 (8:1 DCM-MeOH). [α]_{D} -11.2 (c 1.0 in DMSO). ¹H NMR (300 MHz, (CD₃)₂SO) δ 8.14 (bt, 1 H, *J* = 5.0 Hz, NH), 8.03 (d, 1 H, *J*_{NH,1} = 7.1 Hz, NH), 5.97 (bs, 1 H, H-1), 5.56 (d, 1 H, *J*_{OH,4} = 4.7 Hz, OH), 5.46 (d, 1 H, *J*_{OH,2} = 3.8 Hz, OH), 5.15 (d, 1 H, *J*_{OH,3} = 3.9 Hz, OH), 4.33 (t, 1 H, *J*_{6a,6b} = *J*_{5,6a} = 8.3 Hz, H-6a), 4.15 (dd, 1 H, *J*_{5,6b} = 3.0 Hz, H-6b), 3.55-3.33 (m, 5 H, H-2, H-3, H-5, NHC*H*₂), 3.12 (td, 1 H, *J*_{3,4} = *J*_{4,5} = 8.6 Hz, H-4), 1.55-1.41 (m, 2 H, NHCH₂C*H*₂), 1.33-1.18 (m, 26 H, CH₂), 0.86 (t, 3 H, ³*J*_{H,H} = 6.9 Hz, CH₃). ¹³C NMR (75.5 MHz, (CD₃)₂SO) δ 183.8 (C=Se), 155.6 (CO), 73.4-73.3 (C-4, C-3), 70.3 (C-2), 65.9 (C-6), 64.9 (C-1), 54.2 (C-5), 47.0 (NHCH₂), 31.7-22.5 (CH₂), 14.4 (CH₃). ESIMS: *m*/*z* 536.38 [M + H]⁺.

### EXAMPLE 33. Synthesis of compound 33.

To a solution of isothiocyanate **39** (100 mg, 0.27 mmol) in toluene (3 mL), dodecanol (90 µL) was added and the reaction mixture was stirred overnight at 110 ºC under reflux. The solvent was evaporated and the residue was purified by column chromatography (1:2 EtOAc-cyclohexane) to afford **33.** Yield: 90 mg (60%). *R_{f}* 0.40 (1:1 EtOAc-cyclohexane). [α]_{D} +18.09 (c 1.0 in DCM). ¹H NMR (500 MHz, CDCl₃): 5.32 (t, 1 H, *J*_{1,2} = 6.0 Hz, H-1), 5.03 (m, 1 H, H-3), 4.95 (t, 1 H, *J*_{4,5} = 9.8 Hz, H-4), 4.34 (m, 3 H, CH₂O), 4.19 (dd, 1 H, *J*_{6a,6b} = 9.2 Hz *J*_{5,6b} = 4.4 Hz, H-6b), 3.87 (m, 1 H, H-5), 2.09, 2.00, 1.98 (3 s, 9 H, COCH₃), 1.67-1.19 (m, 20 H, CH₂), 0.81 (t, 3 H, ³*J*_{H,H} = 7.0 Hz, CH₃). ¹³C NMR (75.5 MHz, CDCl₃): δ 191.4 (CS), 169.9, 168.9, 168.8 (CO ester), 154.3 (CO carbamate), 73.3 (C-3), 71.7 (C-4), 69.5 (C-1), 68.0 (C-2), 65.4 (C-6), 51.5 (C-5), 47.0 (CH₂O), 31.9-14.1 (CH₂, CH₃). HRMS (ESI) calcd for [C₂₆H₄₂O₉N₂SNa]⁺ 581.2503; found 581.2496.

### EXAMPLE 34. Synthesis of compound 34.

Compound **34** was obtained by treatment of a solution of **33** (90 mg, 0.162 mmol) in MeOH (2 mL) with 1 M NaOMe in MeOH (48 µL) followed by neutralization with Amberlite^{®} IR120 hydrogen form, filtration, evaporation and purification by colum chromatography (100:10:1 DCM-MeOH-H₂O). Yield: 69 mg (quant.). *R_{f}* 0.70 (100:10:1 DCM-MeOH-H₂O). [α]_{D} +10.21 (*c* 1.0 in MeOH). ¹H NMR (500 MHz, 323 K CDCl₃): 4.51 (m, 1 H, H-1), 4.45 (m, 2 H, CH₂O), 4.33 (dd, 1 H, *J*_{6a,6b} = 9.1 Hz, *J*_{5,6b} = 3.6 Hz, H-6b), 3.68 (m, 1 H, H-5), 3.65 (m, 2 H, H-4, H-2), 3.33 (t, 1 H, *J*_{3,4} = 9.3 Hz, H-3), 1.79-1.33 (m, 20 H, CH₂), 0.93 (t, 3 H, ³*J*_{H,H} = 7.0 Hz, CH₃). ¹³C NMR (125.7 MHz, 323 K, CDCl₃): δ 192.2 (CS), 156.4 (CO carbamate), 73.3 (C-3), 73.0 (C-4), 69.8 (C-1), 68.0 (C-2), 65.8 (C-6), 53.7 (C-5), 47.0 (CH₂O), 31.5-12.9 (CH₂, CH₃). ESIMS: *m*/*z* = 433.37 [M + H]⁺.

### EXAMPLE 35. Stability of the glycolipid mimetics of the invention to the action of glcosidases

The stability of the glyosidic linkage in compounds **2, 4, 6, 8, 10, 12, 14, 16, 18, 20, 22, 25, 27, 29, 32** and **34** selected as representative examples, upon incubation with human fibroblast lysates for 4 h was checked. For such purpose, cells were scraped into ice-cold H₂O (10⁶ cells mL⁻¹) and lysed by sonication. Insoluble materials were removed by centrifugation at 15,000 rpm for 5 min and protein concentrations were determined with Protein Assay Rapid Kit (WAKO, Tokyo, Japan). 10 µL of the lysates in 0.1% Triton X-100 in distilled water were incubated at 37 °C with 20 µL of the compound solution (200 µM) in 0.1 M citrate buffer, pH 4.5. In control experiments, the activity of several lysosomas glycosidases was confirmed using fluorescent substrates. The substrates were 4-methylumbelliferone (4-MU)-conjugated β-D-glucopyranoside (for GCase), α-D-glucopyranoside (for α-glucosidase), α-D-galactopyranoside (for α-Galase) and β-D-galactopyranoside (for β-galactosidase). The liberated 4-methylumbelliferone was measured in the black-well plate with a Perkin Elmer Luminescence Spectrometer (excitation wavelength: 340 nm; emission: 460 nm). No traces of the free polysubstituted 2-oxa-3-oxoindolizidine, which would result from hydrolysis of the glycolipid mimetics, was detected by gas chromatography upon trimethylsilylation, confirming the stability of the compounds to the action of the glycosidases.

**EXAMPLE 36.** Suppression of LPS-induced inflammation (Splenocytes *in vitro).* Splenocytes were treated concurrently with the compounds of the invention and LPS to assess the suppressive function of compounds on LPS-induced inflammation. Selected results are presented in **Figure 1****.** Several of the compounds, particularly compounds **4**, **6**, **14**, **27**, **29** showed prominent inflammation-suppressing function. **EXAMPLE 37.** Suppression on LPS-induced inflammation (BMDM *in vitro).*

Since macrophage is the major responder to LPS-induced inflammation, we assessed the capability of selected compounds to suppress LPS-induced inflammation in bone marrow-derived macrophages (BMDM). Data shown in **Figure 2** demonstrated that compounds **4** and **14** are efficient in suppressing LPS-induced inflammation.

### EXAMPLE 38. Suppression of LPS-induced inflammation in vivo.

To determine the anti-inflammatory function *in vivo,* we assessed the capability of compound **29** in suppressing LPS-induced inflammation in B6 mice. In **Figure 3****,** the level of TNFα in sera was shown to be significantly suppressed when injected twice in a half hour time after LPS-treatment, confirming that compound **29** is efficient in suppressing LPS-induced inflammation.

### EXAMPLE 39. ERK and p38 of the MAPK family and STAT3 are suppressed by the new compounds.

Using the multiplex bead array, we examined how the new compounds might exert their suppressive effect in term of signal transduction. In **Figure 4****,** as a representative example, we found that phosphorylation of ERK and p38 of the MAPK family, but not JNK, was significantly reduced by compounds **4** and **14** comparing to LPS treatment alone. Hence, the new compounds may exercise their anti-inflammation function through suppressing phosphorylation of ERK and p38. We also observed the significant reduction of phosphorylation in STAT3 by compound **14.**

**EXAMPLE 40.** Suppression of αGalCer-induced inflammation (Splenocytes *in vitro).* Splenocytes were treated concurrently with the compounds of the invention and αGalCer to assess their ability to suppress αGalCer-induced inflammation. Selected results are presented in **Figure 5****.** All compounds shown in **Figure 5** showed prominent anti-inflammation function against αGalCer-induced inflammation.

### EXAMPLE 41. Immune-stimulating function of compounds (Splenocytes in vitro).

Splenocytes were treated with the compounds of the invention alone to assess the immune-stimulating function of the compounds by measuring their ability to promote the secretions of IFN-γ and IL-4. In **Figure 6****,** as a representative example, we found that compound **29** was effective in promoting the productions of IFNγ, but not IL-4, supporting that compound **29** is Th1-biased.

### EXAMPLE 42. Suppression of OVA-induced airway hyperreactivity (AHR) and inflammation in vivo.

Using the Th2 OVA-induced AHR mouse model, we assessed the capability of selected compounds **14** and **29** in suppressing Th2 inflammation. We found that compound **14** can suppress AHR onset **(****Figure** 7). Moreover, we observed that compound **29** could significantly suppress the increase of the major instigators of AHR, IL-13 and IL-4, in BALF **(****Figure 8**) and IL-13 in lungs (protein in **Figure 9** and mRNA in **Figure 10**).

### EXAMPLE 43. Evaluating the adjuvancy function of compounds in vivo.

Besides assessing the ability of the new compounds to suppress inflammation, we also looked into their function to act as adjuvants, i.e. their ability to promote desirable immunological trends in the development of vaccines. We measured the *in vivo* adjuvancy capability of selected compounds **29** in inducing IFNγ and IL-4 in B6 mice (**Figure 11**). Compound **29** showed significant capability in inducing both IFNγ and IL-4 2 hours after injection and still having a significant detectable level of IFNγ at 18 hours after injection. This result strongly supports the usage of the compounds of the invention as the adjuvant in vaccine formulation.

## Claims

1. A compound of formula **I:** 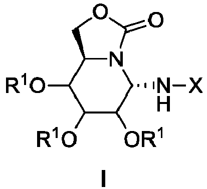 wherein:
each R¹ is independently -H, -CH₂Ph (Bn), -COPh (Bz), -CO-C₁-C₁₂ alkyl, C₁-C₁₂ alkyl, wherein each R¹ is independently optionally substituted by one or more groups R²;
R² is -OH, -N(R³)₂, -N₃, C₁-C₄ alkyl or halogen;
each R³ is independently H or C₁-C₄ alkyl;
X is -C(=S)-OY, -C(=Se)-NHY, -SO₂-NYY', -SO₂-OY or -P(=O)(OY)₂;
Y is -C₁-C₂₅ alkyl optionally substituted by one or more groups R⁴;
Y' is H or -C₁-C₂₅ alkyl optionally substituted by one or more groups R⁴;
R⁴ is -OH, OBn, C₁-C₁₂ alkyl, -O-C₁-C₁₂ alkyl, -S-C₁-C₁₂ alkyl, -N(R³)₂, -N₃, -C(=O)- C₁-C₁₂ alkyl or Cy₁; and
Cy₁ is a 5-membered to 14-membered ring which can be saturated, partially unsaturated or aromatic, and which optionally contains from 1 to 4 identical or different heteroatoms in total selected from N, O, S or P, wherein Cy₁ can be optionally fused to a 5- or 6-membered carbocycle or heterocycle which can be saturated, partially unsaturated or aromatic, and wherein Cy₁ is optionally substituted by one or more groups selected from phenyl, halogen, C₁-C₄ alkyl, halo-C₁-C₄alkyl, -O-C₁-C₄alkyl, - OCOO-C₁-C₄ alkyl, -CO-C₁-C₄ alkyl, S-C1-C4 alky or N₃-C₁-C₄ alkyl.

2. The compound of formula I according to claim 1, wherein each R¹ is independently selected from -H, -Bn, -Bz, -CO-C₁-C₄ alkyl and -C₁-C₄ alkyl and wherein each R¹ is independently optionally substituted by one or more groups R².

3. The compound of formula **I** according to any of claims 1 or 2, wherein X is selected from -C(=S)-OY, -C(=Se)-NHY, -SO₂-NYY', -SO₂-OY or -P(=O)(OY)₂.

4. The compound of formula **I** according to any of claims 1 to 3, wherein Y is -C₂-C₁₈ alkyl optionally substituted by one or more groups R⁴.

5. The compound of formula **I** according to any of claims 1 to 4, wherein Y' is H or - C₂-C₁₈ alkyl optionally substituted by one or more groups R⁴.

6. The compound of formula I according to claim 1, which is selected from:
1 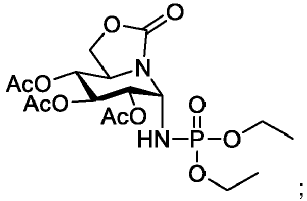
2 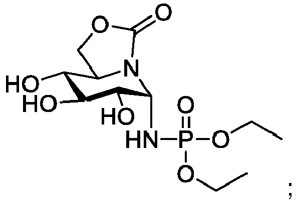
3 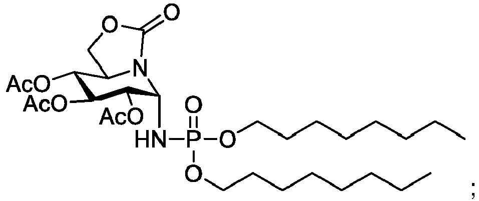
4 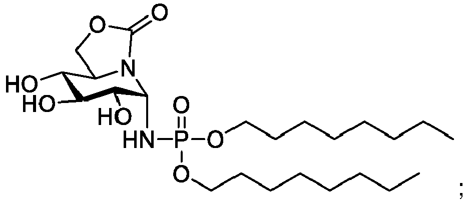
5 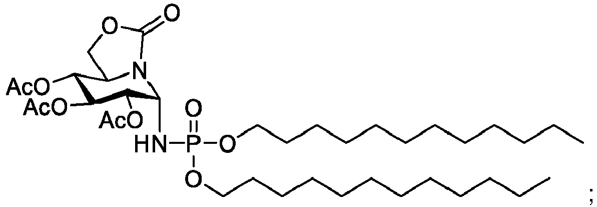
6 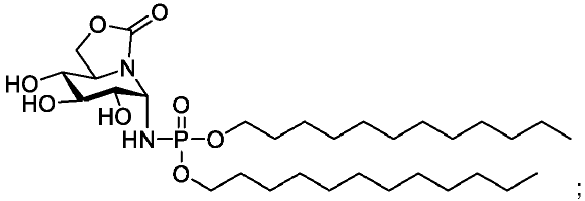
7 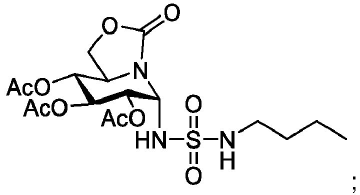
8 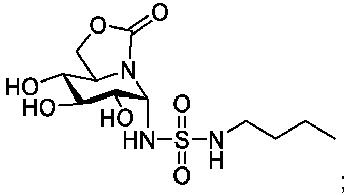
9 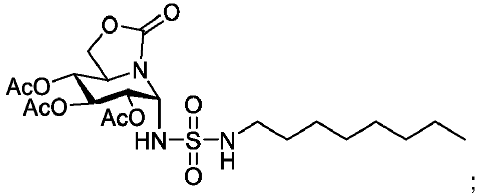
10 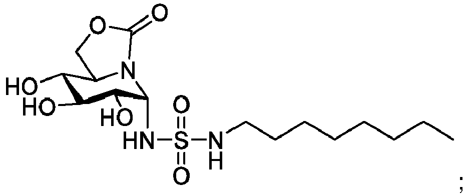
11 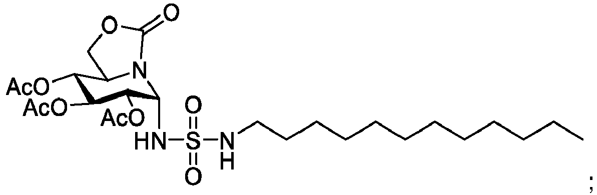
12 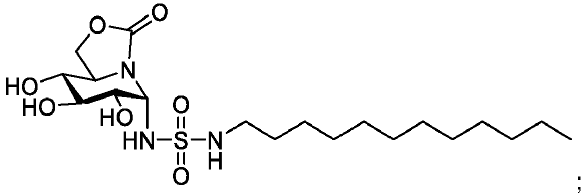
13 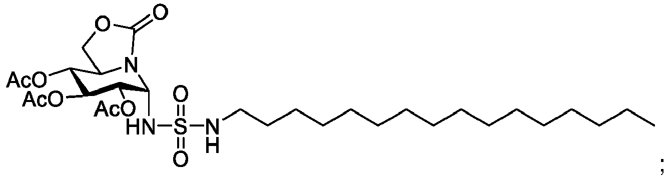
14 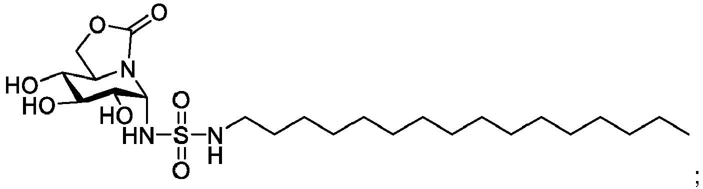
15 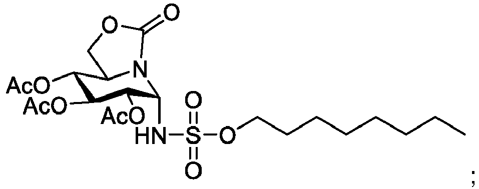
16 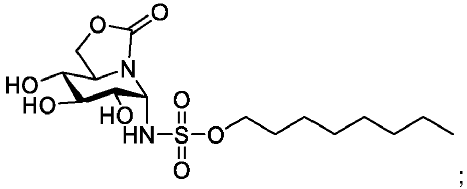
17 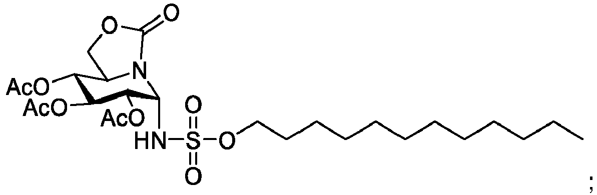
18 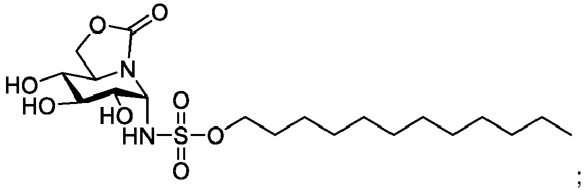
19 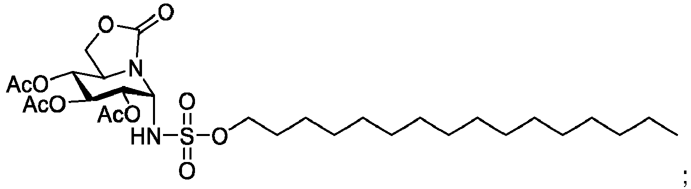
20 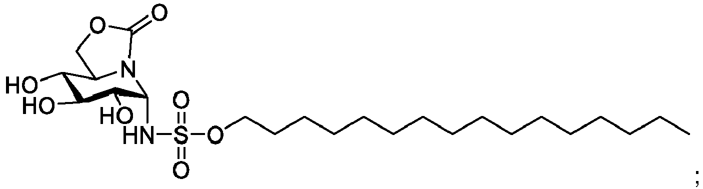
24 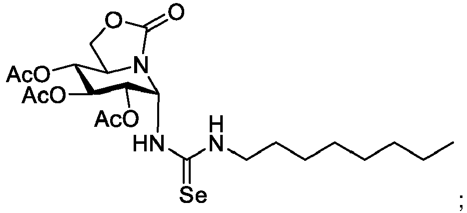
28 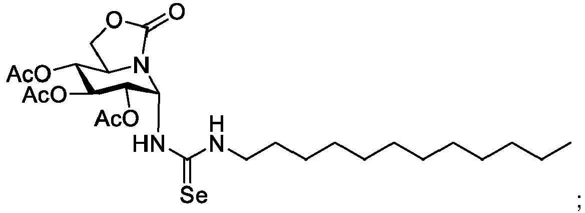
29 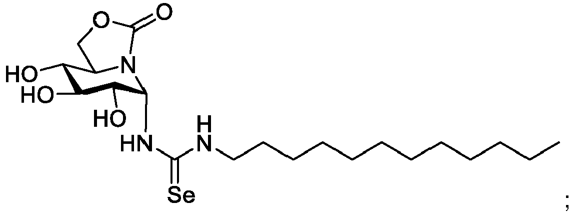
31 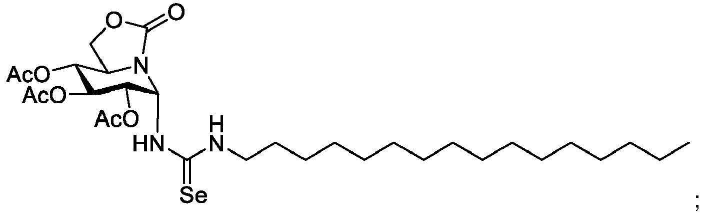
32 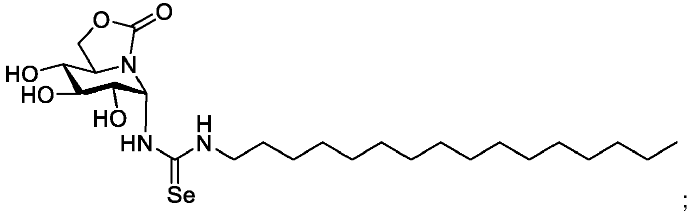
33 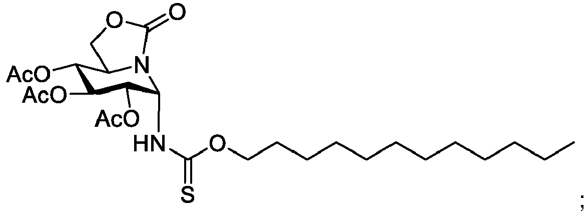 and
34

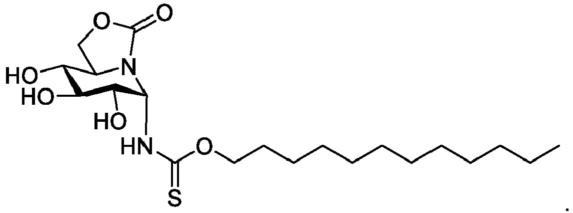

7. A pharmaceutical composition which comprises at least one compound of formula I or any mixtures or combinations thereof.

8. A compound of formula I according to any of claims 1 to 6 or a pharmaceutical composition thereof for use as a medicament.

9. A compound of formula I': 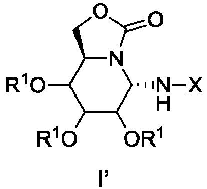 wherein:
each R¹ is independently -H, -CH₂Ph (Bn), -COPh (Bz), -CO-C₁-C₁₂ alkyl, C₁-C₁₂ alkyl, wherein each R¹ is independently optionally substituted by one or more groups R²;
R² is -OH, -N(R³)₂, -N₃, C₁-C₄ alkyl or halogen;
each R³ is independently H or C₁-C₄ alkyl;
X is -C(=S)-OY, -C(=S)-NYY', -C(=O)-NYY', C(=Se)-NHY, -C(=NY)-NHY', -SO₂-Y, - SO₂-NYY', -SO₂-OY or -P(=O)(OY)₂;
Y is -C₁-C₂₅ alkyl optionally substituted by one or more groups R⁴;
Y' is H or -C₁-C₂₅ alkyl optionally substituted by one or more groups R⁴;
R⁴ is -OH, OBn, C₁-C₁₂ alkyl, -O-C₁-C₁₂ alkyl, -S-C₁-C₁₂ alkyl, -N(R³)₂, -N₃, -C(=O)- C₁-C₁₂ alkyl or Cy₁; and
Cy₁ is a 5-membered to 14-membered ring which can be saturated, partially unsaturated or aromatic, and which optionally contains from 1 to 4 identical or different heteroatoms in total selected from N, O, S or P, wherein Cy₁ can be optionally fused to a 5- or 6-membered carbocycle or heterocycle which can be saturated, partially unsaturated or aromatic, and wherein Cy₁ is optionally substituted by one or more groups selected from phenyl, halogen, C₁-C₄ alkyl, halo-C₁-C₄alkyl, -O-C₁-C₄alkyl, - OCOO-C₁-C₄ alkyl, -CO-C₁-C₄ alkyl, S-C1-C4 alky or N₃-C₁-C₄ alkyl,
or a pharmaceutical composition thereof for use in the treatment and/or prevention of an immune disease.

10. The compound of formula I' or a pharmaceutical composition thereof for their use according to claim 9, selected from:
1 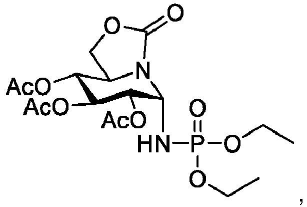
2 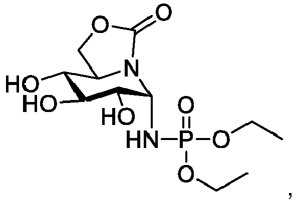
3 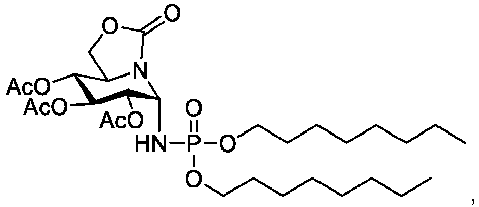
4 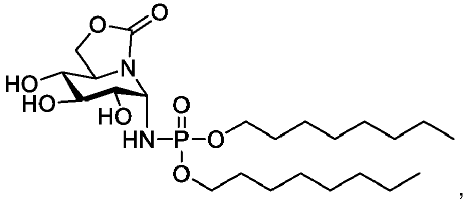
5 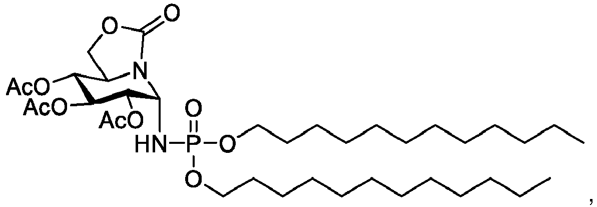
6 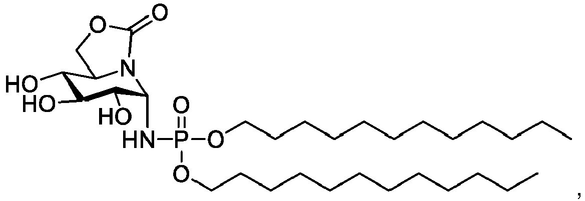
7 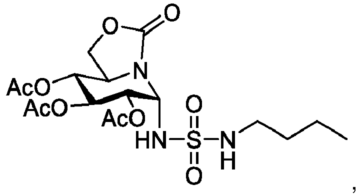
8 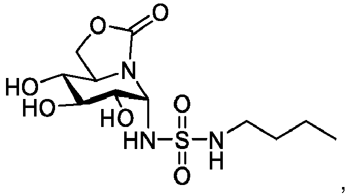
9 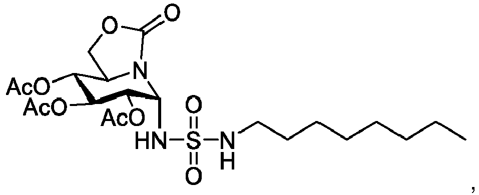
10 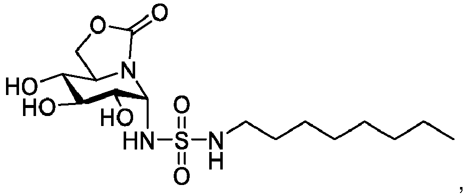
11 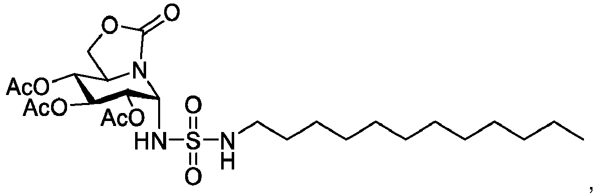
12 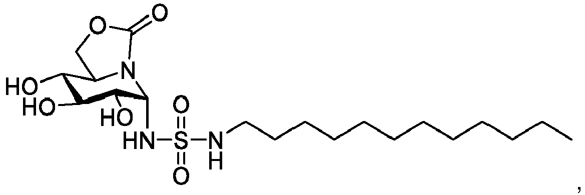
13 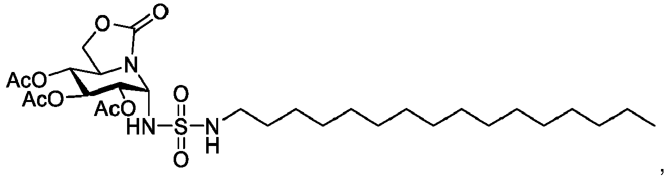
14 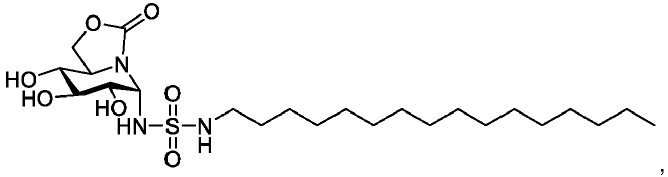
15 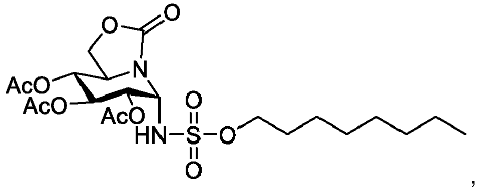
16 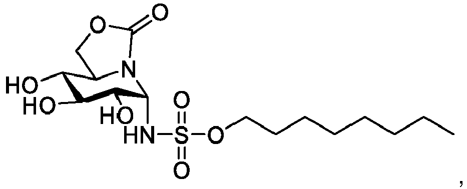
17 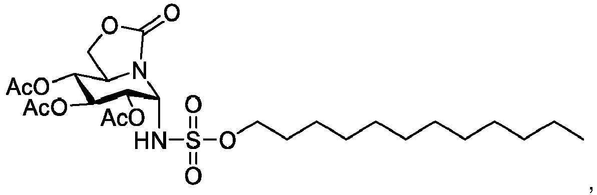
18 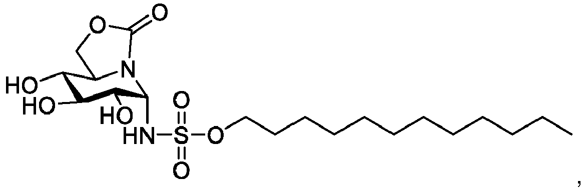
19 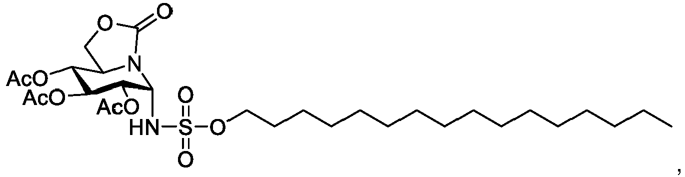
20 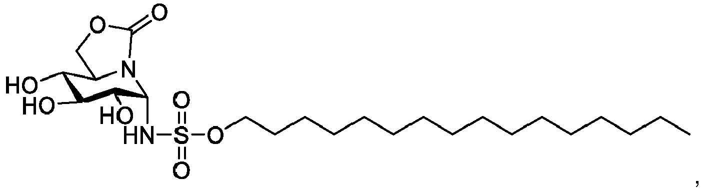
21 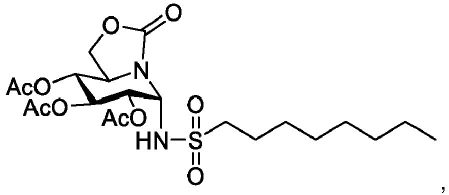
22 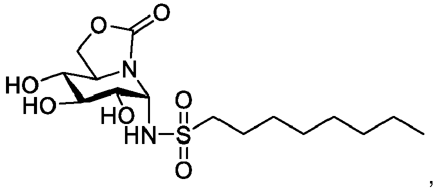
23 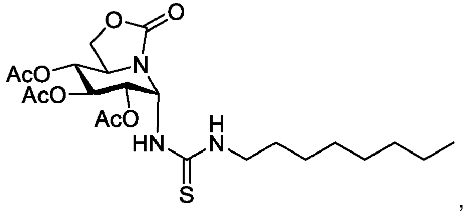
24 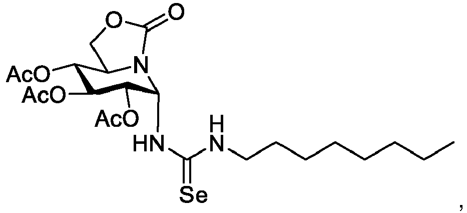
25 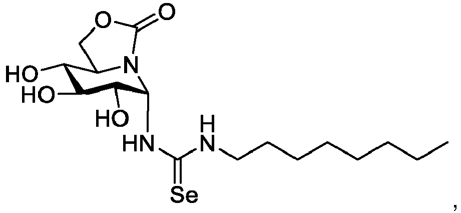
26 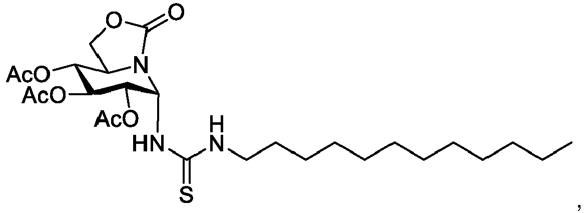
27 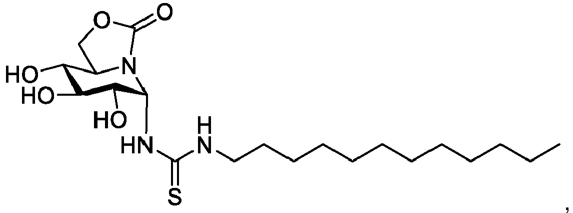
28 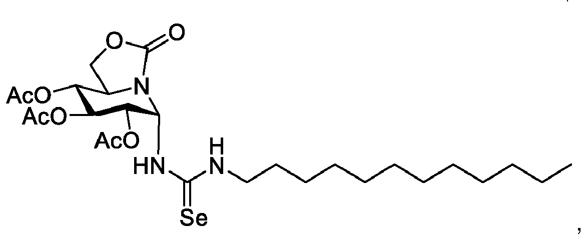
29 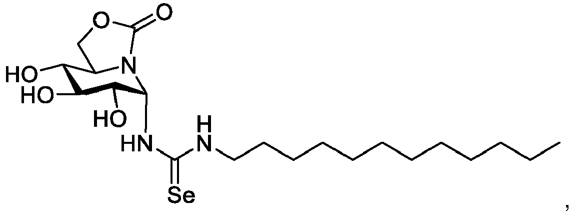
30 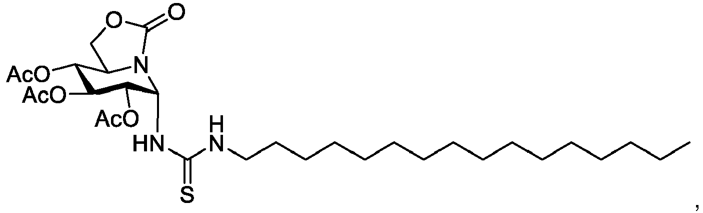
31 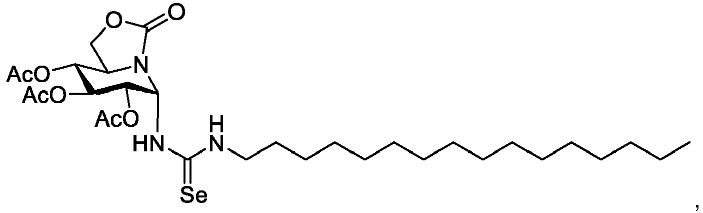
32 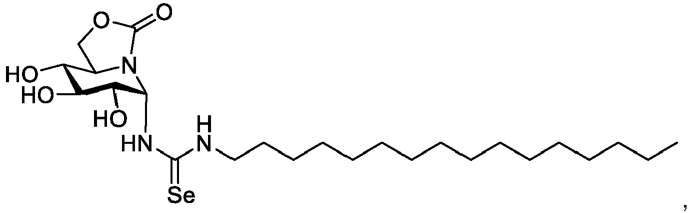
33 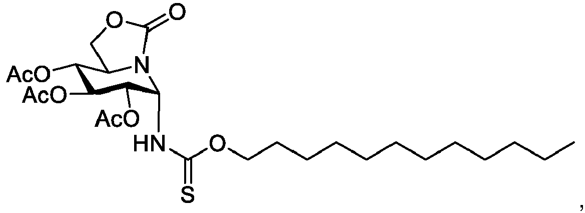 and
34

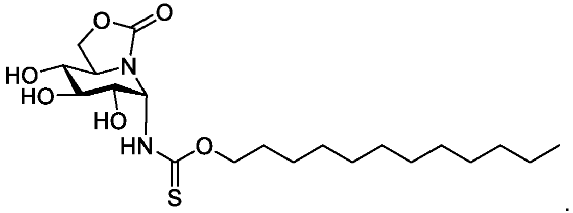

11. The compound of formula I' or the pharmaceutical composition thereof for their use according to any of claims 9 or 10, wherein the immune disease is selected from acute inflammation, chronic disease allergy, cancer chemotherapy, infectious disease, the Metabolic Syndrome, non-alcoholic fatty liver disease (NAFLD), non-alcoholic steatohepatitis (NASH), immune mediated hepatitis, an autoimmune disease, graft rejection pathology, inflammatory bowel disease, atherosclerosis and airway hyperactivity.

12. The compound of formula I' or the pharmaceutical composition thereof for their use according to claim 11, wherein the airway hyperactivity is selected from asthma and allergic rhinitis.
